# EUROPEAN PATENT SPECIFICATION

(11) **EP 3 395 341 B1**
(45) Date of publication and mention of the grant of the patent: **05.02.2025**
(21) Application number: 16877674.8
(22) Date of filing: 16.12.2016
(51) Int. Cl.: A61K 31/198, A61K 31/4415, A61K 31/401, A61P 25/00, A61P 25/28

(54) **COMPOSITION FOR TREATING MOTOR NEURON DISEASES AND USE THEREOF**
ZUSAMMENSETZUNG ZUR BEHANDLUNG VON MOTONEURONENERKRANKUNGEN UND VERWENDUNG DAVON
COMPOSITION POUR LE TRAITEMENT DE MALADIES NEUROMOTRICES ET SON UTILISATION

(30) Priority: 21.12.2015 CN 201510969710; 14.12.2016 CN 201611153606
(43) Date of publication of application: 31.10.2018
(73) Proprietor: Nanjing Jianrong Bio-Technology Co., Ltd., Nanjing, Jiangsu 210032 (CN)
(72) Inventor: YUE, Maoxing, Chaoyang, Beijing 100101 (CN); HUANG, Tongge, Nanjing, Jiangsu 210000 (CN)
(74) Representative: Engelhard, Markus
(86) International application number: PCT/CN2016/110316
(87) International publication number: WO 2017/107863

(56) References cited:
- EP-A1- 2 601 951
- EP-A1- 2 881 112
- EP-A1- 3 165 221
- WO-A2-02/43507
- CN-A- 101 912 394
- CN-A- 102 772 407
- CN-A- 104 055 773
- CN-A- 104 055 773
- CN-A- 107 184 582
- US-A1- 2015 250 884

## Description

### BACKGROUND

### Technical Field

The present invention relates to the pharmaceutical field, and particularly to a composition for treating motor neuron diseases and use thereof.

### Related Art

Motor neuron diseases (MNDs) are a group of chronic, progressive, degenerative diseases with unknown causes that selectively affect the anterior horn cells in spinal cord, the motor neurons in brain stem, the pyramidal cells in cerebral cortex, and the pyramidal tract, and are characterized in pathology that progressive degeneration, necrosis and apoptosis of upper and lower motor neurons. Clinically, the patients have the manifestations of damage of both upper and/(or) lower motor neurons which are different combinations of muscle weakness, muscle atrophy, and pyramidal tract signs, and eventually die of respiratory failure frequently, with the sensory and sphincter functions generally unaffected. Due to the different combinations of symptoms and signs, various types of MNDs are formed, including amyotrophic lateral sclerosis (ALS), spinal muscular atrophy (SMA), primary lateral sclerosis (PLS), and progressive bulbar palsy (PBP), in which ALS is the most common type of chronic motor neuron disease, and also known as "Lou Gehrig's disease".

The etiology and pathogenesis are still unclear at present. The generally accepted etiology and pathogenesis include free radical oxidation theory, excitatory amino acid toxicity, neurotrophic factor disorder, autoimmune mechanism, viral infection and environmental factors. Each hypothesis is supported by some evidences, and the more accepted understanding at present is that on the basis of genetic background, the oxidative damage and excitotoxic effect jointly damage the motor neurons, mainly affecting the structures and functions of mitochondria and cytoskeleton.

There is no clear and effective treatment for "ALS". The average survival time of patients is only 2-5 years. ALS is listed as one of the five terminal illnesses by the World Health Organization. The life of 1 patient with "ALS" is taken away every 90 minutes in the world. According to statistics, by the end of 2010, there are about over 200,000 patients with ALS in China. At present, the treatment measures for MNDs are mainly to relieve the symptoms, delay the progression of the disease and improve the quality of life of the patients. Medications to the disease mainly include Rilutek (Riluzole), the only drug currently approved by the Food and Drug Administration (FDA) for the treatment of ALS, which is a drug that combats the glutamate toxicity, and also the only drug that is currently confirmed to be effective in animal models and effective in clinic, and which can delay the progression of the disease, where the survival time of the patients can only be extended by about six months. Other medications also include Mecobalamin, B vitamins, vitamin E, and a variety of neurotrophic agents. In traditional Chinese medical science, the diseases are categorized as "flaccid paralysis", and the pathogenesis includes weakness of the five internal organs, lack of body fluid, deficiency of qi and blood, malnutrition of muscles and tendons, and weakness of stomach and spleen. Dialectical treatment is employed for the treatment of the diseases, in which acupuncture and medication are given simultaneously, and the treatment is focused on adjusting the functions of spleen and stomach, tonifying and reinforcing the weakness of spleen, and reinforcing the weakness of heart, lung, liver, and kidney to ameliorate the symptoms of, delay the development of, and alleviate the MNDs in the patients.

There is no drug or healthcare product available at present that is safe, effective, and cost-effective for MNDs.

### SUMMARY

An object of the present invention is to provide a composition for treating MNDs.

Another object of the present invention is to provide use of the composition.

The object of the present invention is accomplished through the following technical solutions.

The invention is defined by the appended claims.

A composition for use in a method for treating MNDs is provided, wherein the MNDs comprise amyotrophic, spinal muscular atrophy, primary lateral sclerosis, or progressive bulbar palsy, said composition comprising, in each unit, substances in amounts meeting the following proportional relationship, wherein "unit" refers to the
daily dosage administered to a patient:
0.3 to 8 g of L-ornithine or an L-ornithine salt with an L-ornithine content equivalent to 0.3 to 8 g, 0.5 to 5 g of aspartic acid, and 2 to 20 g of vitamin B₆;
preferably 0.325 to 8 g of L-ornithine or an L-ornithine salt with an L-ornithine content equivalent to 0.325 to 8 g, 0.625 to 5 g of aspartic acid, and 3 to 20 g of vitamin B₆;
further preferably 0.5 to 8 g of L-ornithine or an L-ornithine salt with an L-ornithine content equivalent to 0.5 to 8 g, 1 to 5 g of aspartic acid, and 6 to 20 g of vitamin B₆;
   or
further preferably 0.325 to 0.65 g of L-ornithine or an L-ornithine salt with an L-ornithine content equivalent to 0.325 to 0.65 g, 0.625 to 1.25 g of aspartic acid, and 3 to 10 g of vitamin B₆.

The composition for use further comprises, in each unit, one or more of arginine, isoleucine, leucine, lysine, methionine, phenylalanine, threonine, tryptophan, valine, histidine, glycine, alanine, proline, asparagine, cysteine, glutamic acid, serine, tyrosine, vitamin B₁, vitamin B₂, vitamin B₃, pantothenic acid, biotin, folic acid, vitamin B₁₂, vitamin C, and KCl.

The amino acids may be replaced by various soluble salts or derivatives thereof.

For example, the lysine is replaced by lysine acetate, the cysteine is replaced by N-acetyl-L-cysteine, and the tyrosine is replaced by N-acetyl-L-tyrosine.

The any one or more substances are used in amounts meeting the following proportional relationship.

The amino acids are used in amounts meeting the proportional relationship of 2.0-10 g of arginine, 2.0-10 g of isoleucine, 3.0-15 g of leucine, lysine acetate with a lysine content equivalent to 1.5-10 g, 0.2-3 g of methionine, 0.3-3 g of phenylalanine, 1.0-10 g of threonine, 0.3-3 g of tryptophan, 2.5-15 g of valine, 1.0-8 g of histidine, 1.5-8 g of glycine, 2.0-10 g of alanine, 1.5-8 g of proline, 0.1-3 g of asparagine, N-acetyl-L-cysteine with a cysteine content equivalent to 0.1-3 g, 1.0-10 g of glutamic acid, 0.5-5 g of serine, and N-acetyl-L-tyrosine with a tyrosine content equivalent to 0.1-3 g.

The vitamins are used in amounts meeting the proportional relationship of 1.0-4.0 mg of vitamin B₁, 1.0-4.0 mg of vitamin B₂, 10-40 mg of vitamin B₃, 3.0-10 mg of pantothenic acid, 0.1-0.4 mg of biotin, 0.1-0.8 mg of folic acid, 2.0-12 µg of vitamin B₁₂, and 1.0-6.0 g of vitamin C.

KCl is used in an amount of 5-10 ml of 10% KCl.

Preferably, the amino acids are used in amounts meeting the proportional relationship of 2.2-4.4 g of arginine, 2.2-4.4 g of isoleucine, 3.4-6.8 g of leucine, lysine acetate with a lysine content equivalent to 1.8775-3.755 g, 0.3-0.6 g of methionine, 0.4-0.8 g of phenylalanine, 1.15-2.3 g of threonine, 0.375-0.75 g of tryptophan, 2.65-5.3 g of valine, 1.175-2.35 g of histidine, 1.575-3.15 g of glycine, 2.075-4.15 g of alanine, 1.775-3.55 g of proline, 0.1375-0.275 g of asparagine, N-acetyl-L-cysteine with a cysteine content equivalent to 0.15-0.3 g, 1.425-2.85 g of glutamic acid, 0.925-1.85 g of serine, and N-acetyl-L-tyrosine with a tyrosine content equivalent to 0.175-0.35 g.

The vitamins are used in amounts meeting the proportional relationship of 1.0-4.0 mg of vitamin B₁, 1.0-4.0 mg of vitamin B₂, 10-40 mg of vitamin B₃, 3.0-10 mg of pantothenic acid, 0.1-0.4 mg of biotin, 0.1-0.8 mg of folic acid, 2.0-12 µg of vitamin B₁₂, and 1.0-6.0 g of vitamin C.

KCl is used in an amount of 5-10 ml of 10% KCl.

Alternatively, preferably, the amino acids are used in amounts meeting the proportional relationship of 3.0-10 g of arginine, 3.0-10 g of isoleucin, 5.0-15 g of leucine, lysine acetate with a lysine content equivalent to 3.0-10 g, 0.5-3 g of methionine, 0.5-3 g of phenylalanine, 3.0-10 g of threonine, 0.5-3.0 g of tryptophan, 5.0-15 g of valine, 3.0-8.0 g of histidine, 3.0-8.0 g of glycine, 3.0-10 g of alanine, 3.0-8.0 g of proline, 0.1-3.0 g of asparagine, N-acetyl-L-cysteine with a cysteine content equivalent to 0.1-3.0 g, 3.0-10 g of glutamic acid, 0.5-5.0 g of serine, and N-acetyl-L-tyrosine with a tyrosine content equivalent to 0.1-3 g.

The vitamins are used in amounts meeting the proportional relationship of 2.0-4.0 mg of vitamin B₁, 2.0-4.0 mg of vitamin B₂, 20-40 mg of vitamin B₃, 6.0-10 mg of pantothenic acid, 0.2-0.4 mg of biotin, 0.2-0.8 mg of folic acid, 4.0-12 µg of vitamin B₁₂, and 2.0-6.0 g of vitamin C.

Specifically, the composition comprises, in each unit, substances in amounts meeting the following proportional relationship: 2.2 g of isoleucine, 3.4 g of leucine, 2.65 g of lysine acetate (equivalent to 1.8775 g of lysine), 0.3 g of methionine, 0.4 g of phenylalanine, 1.15 g of threonine, 0.375 g of tryptophan, 2.65 g of valine, 2.2 g of arginine, 1.175 g of histidine, 1.575 g of glycine, 2.075 g of alanine, 1.775 g of proline, 0.625 g of aspartic acid, 0.1375 g of asparagine, 0.2 g of N-acetyl-L-cysteine (equivalent to 0.15 g of cysteine), 0 or 1.425 g of glutamic acid, 0.415 g of L-ornithine hydrochloride (equivalent to 0.325 g of L-ornithine ), 0.925 g of serine, 0.215 g of N-acetyl-L-tyrosine (equivalent to 0.175 g of tyrosine), 3.0-5.0 g of vitamin B₆, 0 or 5-10 ml of 10% KCl, and 0 or 2.0-6.0 g of vitamin C; or 4.4 g of isoleucine, 6.8 g of leucine, 5.3 g of lysine acetate (equivalent to 3.775 g of lysine), 0.6 g of methionine, 0.8 g of phenylalanine, 2.3 g of threonine, 0.75 g of tryptophan, 5.3 g of valine, 4.4 g of arginine, 2.35 g of histidine, 3.15 g of glycine, 4.15 g of alanine, 3.55 g of proline, 1.25 g of aspartic acid, 0.275 g of asparagine, 0.4 g of N-acetyl-L-cysteine (equivalent to 0.3 g of cysteine), 0 or 2.85 g of glutamic acid, 0.83 g of L-ornithine hydrochloride (equivalent to 0.65 g of L-ornithine), 1.85 g of serine, 0.43 g of N-acetyl-L-tyrosine (equivalent to 0.35 g of tyrosine), 3.0-10 g of vitamin B₆, 0 or 5-10 ml of 10% KCl, and 0 or 2.0-6.0 g of vitamin C.

Any one of the compositions for use may further comprises a suitable amount of 5% glucose and sodium chloride injection (5% GNS), 0.9% sodium chloride injection (0.9% NS), or 10% glucose injection.

For the composition for use comprising glucose, insulin may be further added in an amount of one unit (1u) of insulin per 3-5 g of glucose, and preferably one unit (1u) of insulin per 4 g of glucose.

Still further, in a preferred embodiment of the composition for use, the composition for use has a formula comprising specifically:
250 ml of 0.9% sodium chloride injection + 3.0 g or 5.0 g of vitamin B₆ + 250 ml or 500 ml of compound amino acid injection;
250 ml of 0.9% sodium chloride injection + 3.0 g or 5.0 g of vitamin B₆ + 250 ml or 500 ml of compound amino acid injection + 2.0 g of vitamin C;
250 ml of 0.9% sodium chloride injection + 3.0 g or 5.0 g of vitamin B₆ + 250 ml or 500 ml of compound amino acid injection + 5 ml of 10% potassium chloride;
250 ml of 0.9% sodium chloride injection + 3.0 g or 5.0 g of vitamin B₆ + 250 ml or 500 ml of compound amino acid injection + 2.0 g of vitamin C + 5 ml of 10% potassium chloride;
250 ml of 5% glucose and sodium chloride injection + 4u insulin injection + 3.0 g or 5.0 g of vitamin B₆ + 2.0 g of vitamin C + 5 ml of 10% potassium chloride + 250 ml of compound amino acid injection; or
250 ml of 10% glucose injection + 8u insulin injection + 3.0 g or 5.0 g of vitamin B₆ + 2.0 g of vitamin C + 5 ml of 10% potassium chloride + 250 ml of compound amino acid injection,
where
the compound amino acid injection comprises, in every 1000 ml, 8.80 g of isoleucine, 13.60 g of leucine, 10.60 g of lysine acetate (equivalent to 7.51 g of lysine), 1.20 g of methionine, 1.60 g of phenylalanine, 4.60 g of threonine, 1.50 g of tryptophan, 10.60 g of valine, 8.80 g of arginine, 4.70 g of histidine, 6.30 g of glycine, 8.30 g of alanine, 7.10 g of proline, 2.50 g of aspartic acid, 0.55 g of asparagine, 0.80 g of N-acetyl-L-cysteine (equivalent to 0.60 g of cysteine), 5.70 g of glutamic acid, 1.66 g of L-ornithine hydrochloride (equivalent to 1.30 g of L-ornithine), 3.70 g of serine, and 0.86 g of N-acetyl-L-tyrosine (equivalent to 0.70 g of tyrosine).

Any one of the compositions for use is in any dosage form selected from injectable solutions, oral liquids, tablets, granules, capsules, and instant soluble powders. A corresponding dosage form may contain commonly used excipients suitable for the dosage form, such as water, magnesium stearate, and dextrin, etc, which are well known to those skilled in the art.

Use of any of the compositions in the preparation of drugs or healthcare products for treating MNDs is also provided. The MNDs comprise amyotrophic lateral sclerosis, spinal muscular atrophy, primary lateral sclerosis, or progressive bulbar palsy, and preferably amyotrophic lateral sclerosis.

A method for treating MND is also disclosed, but does not form part of the invention. Such method comprises giving the composition to a patient by injection or oral administration, where the injection is preferably intravenous injection.

In the method, the patient is given the composition by injection or oral administration for 10-40 days and preferably 15-30 days in each course of treatment, and wherein, optionally, after one course of treatment is ended, a next course of treatment is proceeded after an appropriate break time (for example, 10-15 days) as desired.

Further, a glucose injection and insulin may be supplemented for patients with dysphagia, cough, or eating problem. It should be noted that as is clinically observed, the treatment effect is not obvious if the daily dose of vitamin B₆ is less than 1.0 g.

In addition, the treatment effect of direct intravenous bolus injection of undiluted vitamin B₆ is not significant.

The contents of the substances in each unit of the composition according to the present invention may also be expressed in parts by weight, without affecting the proportional relationship of the content of each substance in the composition. The each unit generally refers to the daily dosage administered to patient, and can be appropriately adjusted according to the condition. When prepared into different dosage forms, the daily dosage can be made into a package such as a vial or a bag of injectable solution, or into several basic units as desired, such as into several tablets.

The amino acids in the present invention are preferably L-amino acids.

The mechanism of treatment for MNDs with the composition of the present invention is discussed.

The resting potential refers to the potential difference between the inner and outer sides of the cell membrane when the cell is not stimulated (in a resting state). As is well known, resting potential exists in nerve and muscle fibers. J. Bernstein proposed for the first time in 1902 that the K⁺ distribution on both sides of the neuronal membrane is unequal and the selective permeability of the membrane to the potassium ion is the cause of the resting potential. Resting nerve cells are highly permeable to K⁺, and less permeable to Na⁺. It is the selective permeability that creates a K⁺ concentration gradient across the membrane and creates a transmembrane potential difference. It can be seen that the uneven distributions of ions at the inner and outer sides of the cell membrane and the different permeability to different ions in a quiescent state are the prerequisites for the generation of resting potential. The extracellular Na⁺ concentration is higher than the intracellular Na⁺ concentration, and the intracellular K⁺ concentration is much higher than the extracellular K⁺ concentration, so there is a difference in ion concentrations at both sides of the cell membrane. In addition, there is also a potential difference. The diffusion kinetics of ions depends on the concentration difference and the potential difference. In a quiescent state, the cells are only permeable to K⁺ and quite less permeable to Na⁺ and other ions. K⁺ diffuses out of the membrane as driven by the concentration difference. Since K⁺ is a positive ion, an outer-positive-to-inner-negative potential difference resulting from the outward diffusion will prevent the further diffusion of K⁺. When the driving force of the concentration difference driving K⁺ to flow outward is equal to the resisting force of the potential force preventing K⁺ from flowing out, the outflow of K⁺ is stopped, upon which the transmembrane potential is called the K⁺ equilibrium potential. The resting potential level is mainly affected by the following factors. (1) The extracellular K⁺ concentration affects the resting potential level. If the extracellular K⁺ concentration increases and the intracellular K⁺ concentration difference decreases, then the outflow of K⁺ will decrease, and the resting potential will also decrease. (2) The relative permeability of the membrane to K⁺ and Na⁺ affect the resting potential level. If the permeability of the membrane to K⁺ increases, then the resting potential will increase, and if the permeability of the membrane to Na⁺ increases, the resting potential will decrease. (3) The level of sodium pump activity affects the resting potential.

Action potential refers to a rapidly expandable potential change produced on the basis of the resting potential when excitable cells are stimulated. The mechanism underlying the production of action potential is that the extracellular Na⁺ concentration is higher than intracellular Na⁺ concentration, and thus Na⁺ has a tendency of flowing inward along the concentration difference. In a quiescent state, the permeability of the cell membrane to Na⁺ is small, and the inflow of Na⁺ is thus very low. When the cells are stimulated, a small number of Na⁺ channels on the cell membrane are activated and opened, Na⁺ flows in along the concentration difference and the potential difference, the negative potential at the inner side of the membrane gradually becomes smaller, and depolarization occurs. When the depolarization reaches a certain degree, a large number of sodium channels on the membrane are activated and opened, and a large number of Na⁺ flow in, causing the action potential to burst. The critical membrane potential at which the permeability of the membrane to Na⁺ is increased suddenly is called the threshold potential. The threshold potential is about 10-20 mv smaller than the resting potential, and the threshold potential of nerve cells is about -55 mv. Stimulation can cause the burst of action potential only when the negative potential at the inner side of the membrane is reduced to the threshold potential. Since Na⁺ diffuses along the concentration difference and the potential difference, Na⁺ flows in at a high rate, such that the negative potential at the inner side of the membrane declines and disappears rapidly, and further turns into a positive potential. The positive potential formed at the inner side of the membrane creates an electrical field-induced resisting force to the inflow of Na⁺, to prevent the inflow of Na⁺. When the force (concentration difference) driving Na⁺ to flow in is equal to the force (electrical field-induced resisting force) preventing Na⁺ from flowing in, the inflow of Na⁺ is stopped, upon which the action potential reaches the maximum amplitude, that is, the electric-chemical equilibrium potential of Na⁺, whereby a rising phase of the action potential is formed. The opening time of the sodium channels is short, and the sodium channels are inactivated and closed quickly. At this time, the permeability of the cell membrane to K⁺ increases, and a large number of K⁺ flow out rapidly along the concentration difference and the potential difference, such that the potential at the inner side of the membrane changes from a positive value to a negative value, repolarization occurs, and returns to the level in the resting state, forming a falling state of the action potential. In short, the rising phase of the action potential is the electric-chemical equilibrium potential formed by the inflow of Na⁺, and the falling branch of the action potential is the electric-chemical equilibrium potential formed by the outflow of K⁺. After an action potential occurs to the cell, the membrane potential basically returns to the level in the resting state, but the ion distribution does not. There is too much K⁺ outside the cell, and there is too much Na⁺ in the cell. At this time, the sodium pump on the cell membrane is activated. The sodium pump acts to pump excess extracellular K⁺ into the cell and pump excess intracellular Na⁺ out of the cell to restore the previous levels in the resting state and to make preparations for the next excitation.

Amino acids are the most essential substances that constitute the proteins in and correlates with the life activities of living organisms. They are the basic units that constitute protein molecules in and are closely related to the life activities of living organisms. Amino acids have special physiological functions in antibodies and are one of the essential nutrients in organisms. Through metabolism, amino acids play a role in human in (1) synthesis of proteins and nucleic acids; (2) conversion into acids, hormones, antibodies, creatine and other nitrogenous substances; (3) conversion into carbohydrates and fats; and (4) oxidation into carbon dioxide, water and urea, to generate energy.

L-ornithine is a non-proteinogenic amino acid, which is mainly involved in the urea cycle in organisms and plays an important role in the excretion of ammonia nitrogen from the body. Carbamoyl phosphate synthase I is an enzyme involved in the urea cycle and is present in the mitochondria, where ammonia is used as a nitrogen source, N-acetylglutamate is needed, and the resulting carbamyl phosphate is used to synthesize a urea. Carbamoyl phosphate synthase II is present in the cytosol, where glutamine is used as a nitrogen source, and N-acetylglutamic acid is not needed, and the synthesized carbamyl phosphate is used to synthesize pyrimidines. The first step of reaction catalyzed by the carbamoyl phosphate synthase I is a rate limiting step in the urea cycle. The carbamoyl phosphate synthase I is activated by N-acetylglutamate allosterase. This metabolite is synthesized from glutamate and acetyl CoA under the catalysis of N-acetylglutamate synthase. When the degradation rate for amino acids is increased, as a result of transamination, the concentration of glutamate increases accordingly, and the increase in glutamate concentration promotes the synthesis of N-acetylglutamate, resulting in the activation of carbamoyl phosphate synthase, so the urea synthesis is accelerated. Therefore, excess nitrogen produced by amino acid degradation is efficiently excreted from the body. Since arginine is an activator for N-acetylglutamate synthase, an increase in arginine concentration also accelerates the synthesis of urea.

Aspartic acid promotes the tricarboxylic acid cycle by deamination to produce oxaloacetic acid, so aspartic acid is an important component in the tricarboxylic acid cycle. Aspartic acid also plays an important role in ornithine cycle and nucleotide synthesis, and is a precursor for synthesizing various amino acids and purines and pyrimidine bases. It has strong affinity for cells, and can be used as a carrier for potassium and magnesium ions to deliver electrolytes to the myocardium, to promote cell depolarization, maintain myocardial contractility, reduce myocardial oxygen consumption, and exert a protective effect on myocardium in the case of hypoxia caused by coronary circulation disorder. That is, aspartic acid can directly participate in the urea cycle and in the tricarboxylic acid cycle and the synthesis of nucleic acids in hepatocytes, which is beneficial to repairing damaged hepatocytes. In addition, since aspartic acid indirectly promotes the metabolism by tricarboxylic acid cycle in hepatocytes, and provides the intermediate products of energy metabolism, aspartic acid promotes the energy production in hepatocytes, and allows various functions of damaged hepatocytes to restore rapidly. Therefore, the use of L-ornithine and aspartic acid in combination can stimulate the activity of hepatic urea cycle and promote the synthesis of glutamine, thereby greatly enhancing the detoxification function of the liver, rapidly reducing the blood ammonia, and promoting the repair and regeneration of liver cells. Therefore, the presence of amino acids in the human body not only provides an important raw material for the synthesis of proteins, but also provides a material basis for promoting the growth and normal metabolism, and sustaining life.

B vitamins are water soluble vitamins, and include vitamin B₁, vitamin B₂, vitamin B₆, vitamin B₁₂, nicotinic acid, pantothenic acid, folic acid, and others, which are indispensable substances in promoting the in-vivo metabolism of carbohydrates, fat, and proteins into heat. They synergize to regulate the metabolism, promote the immune system and promote the cell growth and division. vitamin B₆ is probably the most important one of all the B vitamins. vitamin B₆ is also known as pyridoxine, which includes pyridoxol, pyridoxal and pyridoxamine. Pyridoxol can be converted into pyridoxal or pyridoxamine in animal tissues, and vitamin B₆ is mostly present in animal tissues as pyridoxal and pyridoxamine. Pyridoxal and pyridoxamine are both interconvertible with pyridoxal phosphate and pyridoxamine phosphate, and finally present in the tissues and participate in transamination in the form of highly active pyridoxal phosphate and pyridoxamine phosphate. In general, the vitamin B₆ present in muscles accounts for 70-80% of the total amount in human body. vitamin B₆ plays a key role in the metabolism of proteins, lipids and carbohydrates. Therefore, amino acid metabolism disorders may occur to those with large depletion of vitamin B₆. vitamin B₆ is an important coenzyme for the metabolism and synthesis of amino acids, and participates in the physiological processes such as metabolism of unsaturated fatty acids. It is a coenzyme for many important enzyme systems in an organism, and is a nutrient necessary for the normal development of animals and the proliferation of bacteria and yeasts. In addition, vitamin B₆ is also a natural diuretic. Diuresis means detoxification, and intravenous infusion of 5 g vitamin B₆ can lead to an urination of about 380 ml. vitamin B₆ is a coenzyme for amino acid metabolism, particularly for the metabolism of glutamate (Glu) into the neurotransmitter γ-aminobutyric acid (GABA) in human. It is known that the functions of more than 60 enzymes in the liver require the participation of vitamin B₆. Vitamin B₆ plays a very important role in promoting the normal enzymatic metabolism in the body, and has a short half-life and thus can be quickly excreted. Moreover, pyridoxal phosphate also has a special function of promoting the rate of amino acids and potassium entering into the cell.

Potassium is one of the most important inorganic cations in the body. The content of potassium in the electrolytes in the body is only less than that of sodium. 98% of potassium is present in cells, and only 2% is present in extracellular fluids. The serum potassium concentration is 3.5-5.5 mmol/ L. The large difference between intracellular and extracellular potassium concentrations is maintained by the energy-consuming transport of Na⁺-K⁺-ATPase in the cell membrane. Potassium is an important material basis for and involved in the functions of all organs in the body. The physiological functions of potassium include: (1) participating in intracellular carbohydrate and protein metabolism; (2) maintaining intracellular osmotic pressure and regulating acid-base balance; (3) maintaining the resting membrane potential, in which the resting membrane potential mainly depends on the permeability of cell membrane to K⁺ and the difference between K⁺ concentrations at the inner and outer sides of the cell membrane; (4) maintaining the neuromuscular excitability, where a high potassium level increases the neuromuscular excitability, and a low potassium level decreases the excitability; and (5) maintaining the coordination of normal myocardial contraction.

The synaptic transmission mediated by acetylcholine is exemplified. Acetylcholine is an excitatory neurotransmitter on vertebrate neuromuscular junctions (motor endplates). Acetylcholine is synthesized in the axoplasm of the axon terminal of cholinergic neurons. Choline and acetyl-CoA produce acetylcholine under the action of choline acetyltransferase. Choline cannot be synthesized in nerve cells, and are supplied mainly through blood circulation, by taking up into cells by the choline transporter, a specific carrier on the cell membrane. Cholinesters synthesized in the liver, choline stored in glial cells, and choline produced by hydrolysis of acetylcholine released from nerve endings can also be the source of choline in acetylcholine synthesis. Moreover, acetylcholine is packed in synaptic vesicles after synthesis. Each vesicle contains approximately 10³-10⁴ molecules of acetylcholine, and a large number of such synaptic vesicles are present in the vicinity of the plasma membrane of presynaptic axon. The arrival of action potential excites the permeability of presynaptic membrane to Ca²⁺ to increase greatly, and Ca²⁺ flows into the axoplasm along the ion concentration gradient. Due to the increase of intracellular Ca²⁺, the fusion of synaptic vesicles with the plasma membrane is promoted, thereby increasing the release of acetylcholine into the synaptic space. Through this mechanism, in response to one action potential, there may be hundreds of synaptic vesicles that discharge acetylcholine to the synaptic space of a typical neuromuscular synaptic junction. As a result, the local acetylcholine concentration is increased greatly enough to be "sensed" by the acetylcholine receptor, a protein in the plasma membrane of postsynaptic cells. This neurotransmitter binds to many receptor molecules and triggers postsynaptic cells to produce action potentials. Acetylcholine is then rapidly degraded into acetic acid and choline by the acetylcholinesterase in the synaptic space, and the postsynaptic membrane rapidly returns to the resting potential. In this process, the effect of acetylcholine also increases the permeability of the membrane to Na⁺ and K⁺. Because the electrochemical gradient over the postsynaptic membrane is larger than that of K⁺, the inward flow of Na⁺ results in depolarization, causing the transmembrane potential to break down. As long as there is a sufficient number of receptor molecules that bind the neurotransmitter, this local perturbation of the transmembrane potential is sufficient to trigger a new action potential on the neurilemma or sarcolemma where the receptor is located. Thus, the number of receptors occupied at a certain moment governs the inward flow of Na⁺, and the variation in membrane potential thus determined. With the acetylcholinesterase bound to the postsynaptic membrane, acetylcholine is catalytically hydrolyzed quickly, the number of transmitter-receptor complexes is rapidly reduced, and membrane repolarization occurs, to make preparations to receive new action potentials triggering the presynaptic membrane to release more acetylcholine in quantum.

Generally, the resting potential, action potential, generator potential and synaptic potential generated by the neurons all depend on the ion gradient formed by the active transport of the blood-brain barrier, and the transmission and accurate reproduction of nerve impulses also depend on the shielding effect of the blood-brain barrier on neurons and of nerve fibers. Data shows that the glutamate level in cerebrospinal fluid of patients with MNDs are three times higher than that in 80% of the patients in the control group

. The extracellular glutamate has a too high concentration that over stimulates its receptors to produce significant toxic effects on the central nervous system. However, by using the present invention, the glutamate level in the cerebrospinal fluid of some patients is found to be significantly decreased, the motor neurons are repaired, and the patient's symptoms are significantly improved. The underlying reasons include the following. (1) In the present invention, the excess glutamate in the body can react with cysteine and glycine of the present invention to produce glutathione, with which the free radicals in the body are eliminated, and the symptoms of the patients with MNDS are relieved. (2) In the liver, the excess glutamate is oxidatively deaminated to produce α-ketoglutarate and ammonia in the presence of glutamate dehydrogenase (where the coenzyme is NAD⁺ or NADP⁺). Excess ammonia combines with glutamate to produce glutamine, and with glutamine and aspartic acid in the present invention, pyrimidine nucleotides are synthesized in the presence of carbamoyl phosphate synthase II. Moreover, glutamine can also be used, together with the glycine and aspartic acid in the present invention, to synthesize purine nucleotides. Also, glutamate and glycine and aspartic acid in the present invention are involved in the de novo synthesis of purine nucleotides in hepatocyte cytoplasm, providing a source of nucleotides in vivo. By means of the synthesis of pyrimidine nucleotides and purine nucleotides, the protein synthesis is promoted, the damaged neuronal cells are repaired, and the excess ammonia produced by oxidative deamination of glutamate is excreted from the body through urea cycle activated by L-ornithine in the hepatocytes. (3) The glutamine produced can directly penetrate through the blood-brain barrier and decomposed into ammonia and glutamate. Although a too high extracellular glutamate concentration has a significant toxic effect on the central nervous system, the intracellular glutamate is the most abundant excitatory neurotransmitter in the mammalian central nervous system that is involved in the regulation of many important functions of the nervous system, and plays a key role in the neural development, maintenance of synaptic plasticity, formation of neuronal circuits, and learning processes. (4) vitamin B₆ is a natural diuretic, and diuresis means detoxification. Moreover, pyridoxal phosphate can promote the rate of amino acids and potassium entering into the cell. The high dose of vitamin B₆ in the present invention promotes the rate of amino acid entering into neuronal cells, so raw materials and energy are provided for the repair of neurons and the repair of damaged neurons is accelerated. Moreover, K⁺ rapidly entering into neuronal cells promotes the intracellular metabolism of carbohydrate and proteins, regulates the intracellular osmotic pressure and acid-base balance, increases the resting potential of the cell membrane, increases the neuromuscular excitability and makes the normal myocardial contraction coordinated.

In summary, since various amino acids that can provide fresh nutrients for the blood, promote the regeneration of damaged nerve cells through amino acid metabolism and blood circulation, improve the metabolic microenvironment, and enhance the survival of neurons are contained in the present invention, the present invention provides substrates and powerful motivation for metabolism in an organism, to alleviate the condition, and even to reverse the condition of the patients. Furthermore, ornithine and aspartic acid can stimulate the activity of hepatic urea cycle. Through the urea cycle, a large amount of ammonia produced during amino acid metabolism can be excreted. In a living organism, both neuromodulation and hormonal regulation are ultimately enzymatic. Therefore, the large dose of vitamin B₆ added in the present invention provides sufficient coenzymes for the amino acid metabolism, particularly for the metabolism of glutamate (Glu) into the neurotransmitter γ-aminobutyric acid (GABA) in human. Although the glutamate level in the cerebrospinal fluid of patients with MNDs is high, by using the present invention, the glutamate in the cerebrospinal fluid can be converted into glutamine and glutathione. (1) Ammonia can be transported through glutamine from the brain, muscles and other tissues to the liver or kidneys in human. Glutamine is not toxic. It is a way to quickly detoxify ammonia in human. With respect to this disease, it also functions to quickly eliminate the apparent toxic effect of a too high extracellular glutamate concentration on the central nervous system, and to store and transport ammonia. When transported to the liver, glutamine releases ammonia to synthesize urea; when transported to the kidney, glutamine releases ammonia, which is then directly excreted with the urine from the body; and when transported to various tissues, glutamine can be used to synthesize amino acids, purines, pyrimidines, and other nitrogenous substances. In other words, the present invention can promote the synthesis of glutamine, and eliminate the apparent toxic effect of a too high extracellular glutamate concentration on the central nervous system, and glutamine can directly penetrate through the blood-brain barrier and provide some amino acids, purines and pyrimidines for the repair of the nerve cells in brain . (2) The synthesis of glutathione can effectively eliminate free radicals in the body and reduce the symptoms of patients with MNDs. In addition, the amino acids in the present invention also provide energy and raw materials for neurotrophic factors to repair damaged nerve cells. Although a large amount of ammonia is produced through amino acid metabolism in this process, the presence of L-ornithine as a reaction substrate for the urea cycle rapidly activates the urea cycle in the hepatocytes, whereby the harmful ammonia produced in the body is excreted as urea, thus guaranteeing the normal metabolism of the body. Aspartic acid is contained in the present invention, which is a precursor for synthesizing a variety of amino acids and purine and pyrimidine bases, has strong affinity for cells, and can be used as a carrier for potassium and magnesium ions to deliver electrolytes to the myocardium, to promote cell depolarization, and maintain the myocardial contractility. Not only can it provide energy and raw materials for neurotrophic factors to repair damaged nerve cells, but also can promote K⁺ entry into neuronal cells to maintain the myocardial contractility. When used in combination with L-ornithine, aspartic acid can stimulate the activity of the hepatic urea cycle to greatly enhance the detoxification function of the liver, quickly reduce the blood ammonia, and promote the repair and regeneration of liver cells, thereby enhancing the immunity of human, and treating the MNDs radically. The present invention does not have any toxic side effects, and is low in treatment cost, thus being a preferred drug for patients with MNDs.

### The present invention has the following beneficial effects.

The composition provided in the present invention can alleviate or even reverse the progression of MNDs, can basically improve the clinical symptoms of patients with MNDs, and can even allow the patients to have a normal life. The composition has a better therapeutic effect, and is prepared with food-derived raw materials, and thus suitable for long-term use without causing toxic side effects. The composition has the advantages of low cost of treatment, thus basically causing no economic burden to patients with MNDs, and being suitable for clinical promotion.

### BRIEF DESCRIPTION OF THE DRAWINGS

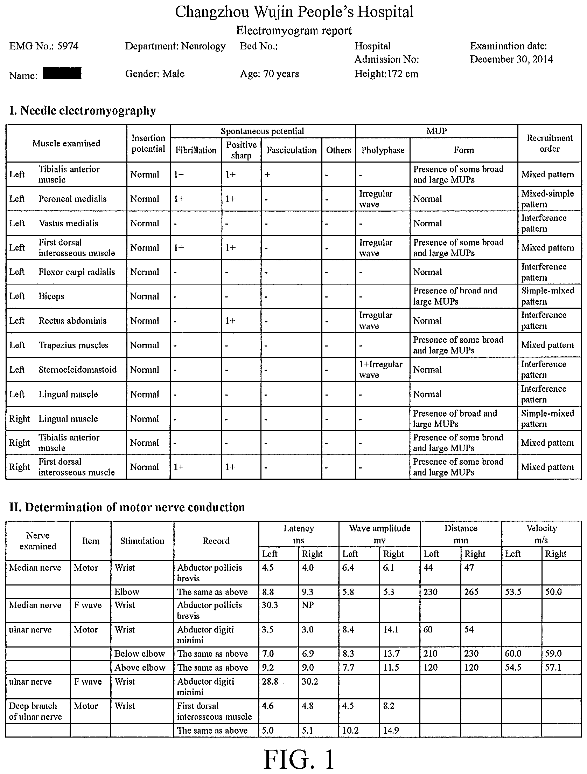

Fig. 1 is an electromyogram report of Case 1.

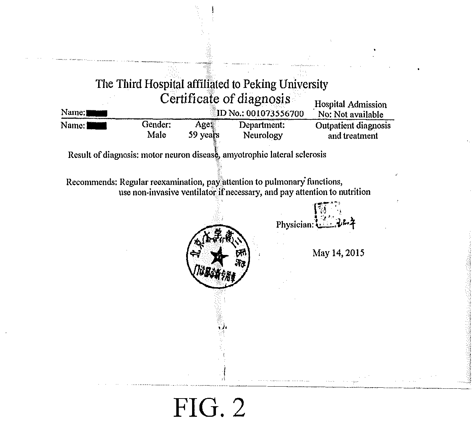

Fig. 2 is a medical certificate for confirmatory diagnosis of ALS in Case 3 by the Third Hospital affiliated to Peking University.

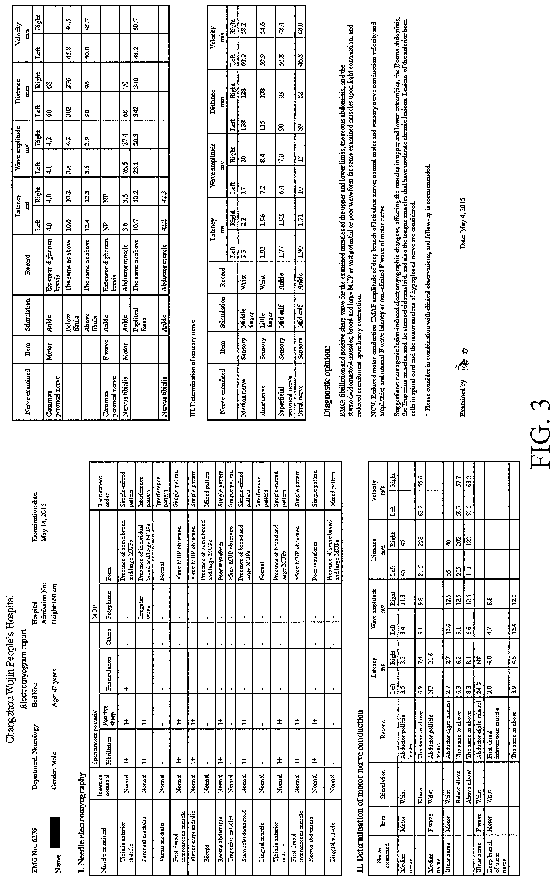

Fig. 3 is an electromyogram report of Case 4.

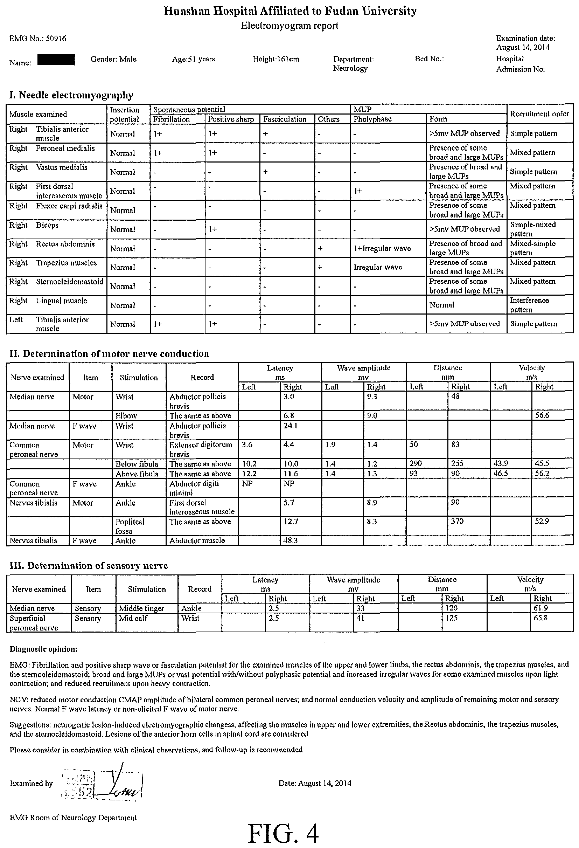

Fig. 4 is an electromyogram report of Case 5.

### DETAILED DESCRIPTION

The present invention is further described with reference to examples below.

### I. Preparation examples of pharmaceutical compositions

General description: The compositions in the examples can be prepared through a conventional process for preparing the corresponding composition.

### Example 1

A pharmaceutical composition was prepared by adding about 3.19 g of L-ornithine hydrochloride (equivalent to 2.5 g of L-ornithine), 1.5 g of aspartic acid, and 10 g of vitamin B₆ to 250 ml of 5% glucose and sodium chloride injection.

### Example 2

A pharmaceutical composition was prepared by adding 0.415 g of L-ornithine hydrochloride (equivalent to 0.325 g of L-ornithine), 0.625 g of aspartic acid, and 3.0 g of vitamin B₆ to 250 ml of 5% glucose and sodium chloride injection.

### Example 3

A pharmaceutical composition was prepared by adding 0.83 g of L-ornithine hydrochloride (equivalent to 0.65g of L-ornithine), 1.25 g of aspartic acid, and 5.0 g of vitamin B₆ to 250 ml of 0.9% sodium chloride injection.

### Example 4

A pharmaceutical composition had a composition comprising 250 ml of a compound amino acid injection [containing 2.2 g of isoleucine, 3.4 g of leucine, 2.65 g of lysine acetate (equivalent to 1.8775 g of lysine), 0.4 g of phenylalanine, 1.15 g of threonine, 0.375 g of tryptophan, 2.2 g of arginine, 1.175 g of histidine, 1.575 g of glycine, 2.075 g of alanine, 1.775 g of proline, 0.625 g of aspartic acid, 0.1375 g of asparagine, 0.2 g of N-acetyl-L-cysteine (equivalent to 0.15 g of cysteine), 0.415 g of L-ornithine hydrochloride (equivalent to 0.325 g of L-ornithine ), 0.925 g of serine, and 0.215 g of N-acetyl-L-tyrosine (equivalent to 0.175 g of tyrosine)], and 3.0 g of vitamin B₆ added to 250 ml of 0.9% sodium chloride injection.

### Example 5

A pharmaceutical composition had a composition comprising 250 ml of a compound amino acid injection [containing 2.2 g of isoleucine, 3.4 g of leucine, 2.65 g of lysine acetate (equivalent to 1.8775 g of lysine), 0.3 g of methionine, 0.4 g of phenylalanine, 1.15 g of threonine, 0.375 g of tryptophan, 2.65 g of valine, 2.2 g of arginine, 1.175 g of histidine, 1.575 g of glycine, 2.075 g of alanine, 1.775 g of proline, 0.625 g of aspartic acid, 0.1375 g of asparagine, 0.2 g of N-acetyl-L-cysteine (equivalent to 0.15 g of cysteine), 1.425 g of glutamic acid, 0.415 g of L-ornithine hydrochloride (equivalent to 0.325 g of L-ornithine ), 0.925 g of serine, and 0.215 g of N-acetyl-L-tyrosine (equivalent to 0.175 g of tyrosine)], and 3.0 g of vitamin B₆ added to 250 ml of 0.9% sodium chloride injection.

### Example 6

A pharmaceutical composition had a composition comprising 250 ml of a compound amino acid injection [containing 2.2 g of isoleucine, 3.4 g of leucine, 2.65 g of lysine acetate (equivalent to 1.8775 g of lysine), 0.3 g of methionine, 0.4 g of phenylalanine, 1.15 g of threonine, 0.375 g of tryptophan, 2.65 g of valine, 2.2 g of arginine, 1.175 g of histidine, 1.575 g of glycine, 2.075 g of alanine, 1.775 g of proline, 0.625 g of aspartic acid, 0.1375 g of asparagine, 0.2 g of N-acetyl-L-cysteine (equivalent to 0.15 g of cysteine), 1.425 g of glutamic acid, 0.415 g of L-ornithine hydrochloride (equivalent to 0.325 g of L-ornithine ), 0.925 g of serine, and 0.215 g of N-acetyl-L-tyrosine (equivalent to 0.175 g of tyrosine)], 5.0 g of vitamin B₆, and 2.0 g of vitamin C added to 250 ml of 0.9% sodium chloride injection.

### Example 7

A pharmaceutical composition had a composition comprising 250 ml of a compound amino acid injection [containing 2.2 g of isoleucine, 3.4 g of leucine, 2.65 g of lysine acetate (equivalent to 1.8775 g of lysine), 0.3 g of methionine, 0.4 g of phenylalanine, 1.15 g of threonine, 0.375 g of tryptophan, 2.65 g of valine, 2.2 g of arginine, 1.175 g of histidine, 1.575 g of glycine, 2.075 g of alanine, 1.775 g of proline, 0.625 g of aspartic acid, 0.1375 g of asparagine, 0.2 g of N-acetyl-L-cysteine (equivalent to 0.15 g of cysteine), 1.425 g of glutamic acid, 0.415 g of L-ornithine hydrochloride (equivalent to 0.325 g of L-ornithine ), 0.925 g of serine, and 0.215 g of N-acetyl-L-tyrosine (equivalent to 0.175 g of tyrosine)], 3.0 g of vitamin B₆, 15 ml of 10% KCl and 2.0 g of vitamin C added to 250 ml of 0.9% sodium chloride injection.

### Example 8

A pharmaceutical composition had a composition comprising 250 ml of a compound amino acid injection [containing 2.2 g of isoleucine, 3.4 g of leucine, 2.65 g of lysine acetate (equivalent to 1.8775 g of lysine), 0.3 g of methionine, 0.4 g of phenylalanine, 1.15 g of threonine, 0.375 g of tryptophan, 2.65 g of valine, 2.2 g of arginine, 1.175 g of histidine, 1.575 g of glycine, 2.075 g of alanine, 1.775 g of proline, 0.625 g of aspartic acid, 0.1375 g of asparagine, 0.2 g of N-acetyl-L-cysteine (equivalent to 0.15 g of cysteine), 1.425 g of glutamic acid, 0.415 g of L-ornithine hydrochloride (equivalent to 0.325 g of L-ornithine ), 0.925 g of serine, and 0.215 g of N-acetyl-L-tyrosine (equivalent to 0.175 g of tyrosine)], 3.0 g of vitamin B₆, 15 ml of 10% KCl and 2.0 g of vitamin C added to 250 ml of 0.9% sodium chloride injection.

### Example 9

A pharmaceutical composition had a composition comprising 250 ml of a compound amino acid injection [containing 2.2 g of isoleucine, 3.4 g of leucine, 2.65 g of lysine acetate (equivalent to 1.8775 g of lysine), 0.3 g of methionine, 0.4 g of phenylalanine, 1.15 g of threonine, 0.375 g of tryptophan, 2.65 g of valine, 2.2 g of arginine, 1.175 g of histidine, 1.575 g of glycine, 2.075 g of alanine, 1.775 g of proline, 0.625 g of aspartic acid, 0.1375 g of asparagine, 0.2 g of N-acetyl-L-cysteine (equivalent to 0.15 g of cysteine), 1.425 g of glutamic acid, 0.415 g of L-ornithine hydrochloride (equivalent to 0.325 g of L-ornithine ), 0.925 g of serine, and 0.215 g of N-acetyl-L-tyrosine (equivalent to 0.175 g of tyrosine)], 5.0 g of vitamin B₆, 15 ml of 10% KCl, 2.0 g of vitamin C, and 4u of insulin injection added to 250 ml of 5% glucose and sodium chloride injection.

### Example 10

A pharmaceutical composition had a composition comprising 500 ml of a compound amino acid injection [containing 4.4 g of isoleucine, 6.8 g of leucine, 5.3 g of lysine acetate (equivalent to 3.775 g of lysine), 0.6 g of methionine, 0.8 g of phenylalanine, 2.3 g of threonine, 0.75 g of tryptophan, 5.3 g of valine, 4.4 g of arginine, 2.35 g of histidine, 3.15 g of glycine, 4.15 g of alanine, 3.55 g of proline, 1.25 g of aspartic acid, 0.275 g of asparagine, 0.4 g of N-acetyl-L-cysteine (equivalent to 0.3 g of cysteine), 2.85 g of glutamic acid, 0.83 g of L-ornithine hydrochloride (equivalent to 0.65 g of L-ornithine ), 1.85 g of serine, and 0.43 g of N-acetyl-L-tyrosine (equivalent to 0.35 g of tyrosine)], and 3.0 g of vitamin B₆ added to 250 ml of 0.9% sodium chloride injection.

### Example 11

A pharmaceutical composition had a composition comprising 500 ml of a compound amino acid injection [containing 4.4 g of isoleucine, 6.8 g of leucine, 5.3 g of lysine acetate (equivalent to 3.775 g of lysine), 0.6 g of methionine, 0.8 g of phenylalanine, 2.3 g of threonine, 0.75 g of tryptophan, 5.3 g of valine, 4.4 g of arginine, 2.35 g of histidine, 3.15 g of glycine, 4.15 g of alanine, 3.55 g of proline, 1.25 g of aspartic acid, 0.275 g of asparagine, 0.4 g of N-acetyl-L-cysteine (equivalent to 0.3 g of cysteine), 2.85 g of glutamic acid, 0.83 g of L-ornithine hydrochloride (equivalent to 0.65 g of L-ornithine ), 1.85 g of serine, and 0.43 g of N-acetyl-L-tyrosine (equivalent to 0.35 g of tyrosine)], 5.0 g of vitamin B₆, 15 ml of 10% KCl and 3.0 g of vitamin C added to 250 ml of 0.9% sodium chloride injection.

### Example 12

A pharmaceutical composition had a composition comprising 500 ml of a compound amino acid injection [containing 4.4 g of isoleucine, 6.8 g of leucine, 5.3 g of lysine acetate (equivalent to 3.775 g of lysine), 0.6 g of methionine, 0.8 g of phenylalanine, 2.3 g of threonine, 0.75 g of tryptophan, 5.3 g of valine, 4.4 g of arginine, 2.35 g of histidine, 3.15 g of glycine, 4.15 g of alanine, 3.55 g of proline, 1.25 g of aspartic acid, 0.275 g of asparagine, 0.4 g of N-acetyl-L-cysteine (equivalent to 0.3 g of cysteine), 2.85 g of glutamic acid, 0.83 g of L-ornithine hydrochloride (equivalent to 0.65 g of L-ornithine), 1.85 g of serine, and 0.43 g of N-acetyl-L-tyrosine (equivalent to 0.35 g of tyrosine)], 3.00 g of vitamin B₆, and 2.0 g of vitamin C added to 250 ml of 0.9% sodium chloride injection.

### Example 13

A pharmaceutical composition had a composition comprising 1000 ml of a compound amino acid injection (containing L-ornithine hydrochloride with an L-ornithine content equivalent to 3.5 g, 2.50 g of aspartic acid, 8.80 g of arginine, 8.80 g of isoleucine, 13.60 g of leucine, lysine acetate with a lysine content equivalent to 7.51 g, 1.20 g of methionine, 1.60 g of phenylalanine, 4.60 g of threonine, 1.50 g of tryptophan, 10.60 g of valine, 4.70 g of histidine, 6.30 g of glycine, 8.30 g of alanine, 7.10 g of proline, 0.55 g of asparagine, N-acetyl-L-cysteine with a cysteine content equivalent to 0.60 g, 5.70 g of glutamic acid, 3.70 g of serine, and N-acetyl-L-tyrosine with a tyrosine content equivalent to 0.70 g), and 8.0 g of vitamin B₆ and 3.0 g of vitamin C, where the vitamins were added to 250 ml of 0.9% sodium chloride injection.

### Example 14

A pharmaceutical composition had a composition comprising 1000 ml of a compound amino acid injection (containing L-ornithine hydrochloride with an L-ornithine content equivalent to 4.5 g, 2.80 g of aspartic acid, 8.30 g of arginine, 6.50 g of isoleucine, 12.00 g of leucine, lysine acetate with a lysine content equivalent to 7.51 g, 1.60 g of methionine, 1.40 g of phenylalanine, 1.80 g of tryptophan, 10.60 g of valine, 4.80 g of histidine, 6.20 g of glycine, 8.50 g of alanine, 7.10 g of proline, 0.55 g of asparagine, 5.70 g of glutamic acid, and 3.70 g of serine, and N-acetyl-L-tyrosine with a tyrosine content equivalent to 0.70 g), 10 g of vitamin B₆, 3 mg of vitamin B₁, 3 mg of vitamin B₂, 40 mg of vitamin B₃, 8 mg of pantothenic acid, 0.4 mg of biotin, 0.6 mg of folic acid, 10 µg of vitamin B₁₂ and 4.0 g of vitamin C.

The vitamins may be directly added to the compound amino acid injection, or to 250 ml of 5% glucose and sodium chloride injection or 0.9% sodium chloride injection.

### Example 15

A pharmaceutical composition had a composition comprising 1000 ml of a compound amino acid injection (containing 8.80 g of isoleucine, 13.60 g of leucine, 10.60 g of lysine acetate (equivalent to 7.51 g of lysine), 1.20 g of methionine, 1.60 g of phenylalanine, 4.60 g of threonine, 1.50 g of tryptophan, 10.60 g of valine, 8.80 g of arginine, 4.70 g of histidine, 6.30 g of glycine, 8.30 g of alanine, 7.10 g of proline, 2.50 g of aspartic acid, 0.55 g of asparagine, 0.80 g of N-acetyl-L-cysteine (equivalent to 0.60 g of cysteine), 5.70 g of glutamic acid, 1.66 g of L-ornithine hydrochloride (equivalent to 1.30 g of L-ornithine ), 3.70 g of serine, and 0.86 g of N-acetyl-L-tyrosine (equivalent to 0.70 g of tyrosine)), and 10 g of vitamin B₆.

The vitamins may be directly added to the compound amino acid injection, or to 250 ml of 5% glucose and sodium chloride injection or 0.9% sodium chloride injection.

### Example 16

A pharmaceutical composition had a composition comprising 1000 ml of a compound amino acid injection (containing 8.80 g of isoleucine, 13.60 g of leucine, 10.60 g of lysine acetate (equivalent to 7.51 g of lysine), 1.20 g of methionine, 1.60 g of phenylalanine, 4.60 g of threonine, 1.50 g of tryptophan, 10.60 g of valine, 8.80 g of arginine, 4.70 g of histidine, 6.30 g of glycine, 8.30 g of alanine, 7.10 g of proline, 2.50 g of aspartic acid, 0.55 g of asparagine, 0.80 g of N-acetyl-L-cysteine (equivalent to 0.60 g of cysteine), 5.70 g of glutamic acid, 1.66 g of L-ornithine hydrochloride (equivalent to 1.30 g of L-ornithine ), 3.70 g of serine, and 0.86 g of N-acetyl-L-tyrosine (equivalent to 0.70 g of tyrosine)), 10 g of vitamin B₆, and 15 ml of 10%KCl.

The vitamins may be directly added to the compound amino acid injection, or to 250 ml of 5% glucose and sodium chloride injection or 0.9% sodium chloride injection.

### Example 17

A health care product as an oral liquid had a composition comprising 1000 ml of a compound amino acid oral liquid (containing 8.80 g of isoleucine, 13.60 g of leucine, 10.60 g of lysine acetate (equivalent to 7.51 g of lysine), 1.20 g of methionine, 1.60 g of phenylalanine, 4.60 g of threonine, 1.50 g of tryptophan, 10.60 g of valine, 8.80 g of arginine, 4.70 g of histidine, 6.30 g of glycine, 8.30 g of alanine, 7.10 g of proline, 2.50 g of aspartic acid, 0.55 g of asparagine, 0.80 g of N-acetyl-L-cysteine (equivalent to 0.60 g of cysteine), 5.70 g of glutamic acid, 1.66 g of L-ornithine hydrochloride (equivalent to 1.30 g of L-ornithine ), 3.70 g of serine, and 0.86 g of N-acetyl-L-tyrosine (equivalent to 0.70 g of tyrosine)), 10 g of vitamin B₆, and 4.0 g of vitamin C. The vitamins may be directly added to the compound amino acid oral liquid, or be packaged separately and orally taken together. 250 ml-1000 ml is orally administered a day.

### Example 17

A healthcare product as a tablet had a composition comprising compound amino acids (including 0.5 g of methionine, 0.5 g of phenylalanine, 1.5 g of threonine, 0.5 g of tryptophan, 3.0 g of valine, 3.0 g of arginine, 1.8 g of glycine, 2.2 g of alanine, 2.0 g of proline, 0.8 g of aspartic acid, 0.5 g of asparagine, 1.5 g of glutamic acid, 3.0 g of L-ornithine hydrochloride, and 1.2 g of serine), and 3.0 g of vitamin B₆, which, together with suitable excipients, are processed into tablets following a conventional tableting process.

Instructions for use of preparations: The above injection preparations containing different doses of compound amino acids (e.g. 250 ml, 500 ml, and 1000 ml) can be selected according to the tolerance and body weight of the patient. The principle is to use a small dose when the patient has gastrointestinal discomfort, and to use a large dose when the patient is well tolerant and has a high body weight. In general, a better effect can be achieved with 250 ml of compound amino acids. In addition, oral preparations may be taken to consolidate or maintain the efficacy after regular infusion therapy.

The content of each substance in above preparation examples is usually the daily dose used by the patient, and can be appropriately adjusted according to the condition of the patient.

### II. Clinical treatment examples

### (I) Clinical observation and statistics

48 patients with motor neuron diseases (amyotrophic lateral sclerosis) were hospitalized, and the clinical treatment was as follows.

### 1. Inclusion of patients

1.1. General condition: 48 patients, aged 40-77 years, more males than females, signed the informed consent form for this therapy when admitted to hospital.

### 1.2. Diagnostic criteria

Patients diagnosed with MNDs or further definitely categorized as amyotrophic lateral sclerosis (ALS) by well-known hospitals (e.g. Peking Union Medical College Hospital, Shanghai Huashan Hospital, the Third Hospital affiliated to Peking University, etc.) in China, and reviewed for confirmatory diagnosis by this hospital based on the Criteria for the Diagnosis of Amyotrophic Lateral Sclerosis of World Federation of Neurology (EEC, 2000) and Chinese Guidelines for Diagnosis and Treatment of Amyotrophic Lateral Sclerosis (2012),
and patients undiagnosed in other hospitals, but diagnosed in this hospital based on the Criteria for the Diagnosis of Amyotrophic Lateral Sclerosis of World Federation of Neurology (EEC, 2000) and Chinese Guidelines for Diagnosis and Treatment of Amyotrophic Lateral Sclerosis (2012), including 36 patients clinically diagnosed as having ALS, 8 patents suspected of having ALS, and 4 patients with clinically probable ALS.

1.3. Exclusion criteria (the patient was excluded once any of the following conditions occurred):
Those who were allergic to the studied drug, are non-cooperative, have serious cardiovascular and cerebrovascular diseases, hepatic and renal dysfunction and other diseases, and receive drugs that are contraindicated and incompatible with the test drug or affect the efficacy of the test drug.

### 1.4. Termination criteria

The patients had severe adverse reactions, and exacerbated symptoms, and other therapies were used; or the patient requested withdrawal for various reasons.

### 2. Therapeutic regimen

The following pharmaceutical composition is given once a day by intravenous infusion for 30 days as a course of treatment:
250 ml of 0.9% sodium chloride injection + 3.0-5.0 g of vitamin B₆ + 250-500 ml of compound amino acid injection. If necessary, 2.0 g of vitamin C and 5 ml of 10% potassium chloride were added separately or simultaneously and formulated into the above pharmaceutical composition for intravenous infusion.

The 0.9% sodium chloride injection might be replaced by 250 ml of 5% glucose and sodium chloride injection or 250 ml of 10% glucose injection as needed by the patients.

Glucose and insulin injections may be supplemented for patients with dysphagia, cough, or eating problem, where the insulin injection was used in an amount of one unit (1u) of insulin per 3-5 g of glucose.

Psychological counseling, life care and symptomatic treatment were strengthened for the patients.

Note: the clinically used compound amino acid injection contained, in every 1000 ml, 8.80 g of isoleucine, 13.60 g of leucine, 10.60 g of lysine acetate (equivalent to 7.51 g of lysine), 1.20 g of methionine, 1.60 g of phenylalanine, 4.60 g of threonine, 1.50 g of tryptophan, 10.60 g of valine, 8.80 g of arginine, 4.70 g of histidine, 6.30 g of glycine, 8.30 g of alanine, 7.10 g of proline, 2.50 g of aspartic acid, 0.55 g of asparagine, 0.80 g of N-acetyl-L-cysteine (equivalent to 0.60 g of cysteine), 5.70 g of glutamic acid, 1.66 g of L-ornithine hydrochloride (equivalent to 1.30 g of L-ornithine), 3.70 g of serine, and 0.86 g of N-acetyl-L-tyrosine (equivalent to 0.70 g of tyrosine).

### 3. Observation indexes for efficacy

3.1. Ineffective: After a course of treatment, no effect was shown or the symptoms continued to deteriorate.

3.2. Effective: After a course of treatment, the patient's condition was controlled, the development of the disease was stopped or the development rate was obviously slower than that before treatment.

3.3. Obviously effective: After one or less than one course of treatment, the patient's condition was improved and either of the following conditions occurred.

3.3.1. The muscle strength at one or more sites was recovered, or the increased muscle tone was gradually reduced.

3.3.2. The patients changed from needing a ventilator to facilitate the breathe to being able to breathe freely, or changed from chronically needing a ventilator to facilitate the breathe to being able to occasionally use a ventilator to facilitate the breathe.

3.3.3. Cough, drooling, dysphagia, sensation of muscle twitch, mobility of the affected area, and other symptoms were obviously ameliorated.

3.3.4. The atrophic muscles grew gradually.

3.3.5. The ALSFRS-R (Amyotrophiclateral sclerosis functional rating scale revised) score was increased.

### Note:

ALSFRS-R is used as a method for assessing the severity of ALS. The scale is simple, easy in operation, and widely used. Its sensitivity, reliability, and stability have been widely confirmed. ALSFRS-R is a scale commonly used in China and other countries for assessing the progression and prognosis of amyotrophic lateral sclerosis, and comprises the items of 1. language; 2. saliva secretion; 3. swallowing; 4. writing; 5. cutting food and use of tableware; 6. dressing and self-care skills; 7. turning and tidying the bedding on the bed; 8. walking; 9. climbing the stairs; 10. difficulty in breathing; 11. orthopnea; and 12. breathing insufficiency. Each item is scored 0 to 4. Functional scores range from 0 (seriously damaged) to 48 (normal).

Scoring is performed by a trained specialized skilled person. Since ALS is a progressive disease, the ALSFRS-R score progressively declines in the absence of effective treatment, and usually has a decline of 5-6 points in 6 months and about 10 points in 12 months.

### 4. Therapeutic effect

After the 48 patients were treated in a one-month course of treatment, the therapy appeared effective in most of the patients after 2 weeks of treatment. After one course of treatment, the therapeutic effects were analyzed. The therapy was shown to be ineffective in 4 cases, effective in 23 cases, and obviously effective in 21 cases.

Note: When the compound amino acid injection was administered at a relatively high dose (e.g. 500 ml/day), a small number of patients had nausea and other symptoms of gastric discomfort. After the dose was reduced (e.g., 250 ml/day), the discomfort disappeared, and the gastric discomfort trended to occur to weaker patients.

### (II) Specific cases

Over 40 patients with MNDs received treatment in this hospital. By using the "combination therapy of compound amino acids with high dose of vitamin B₆", a good therapeutic effect was achieved. Some of the cases were described below.

### Case 1:

Patient: Yang XX, male, aged 70 years. The onset time was April, 2014, and the course of disease had continued for 12 months when the patient was admitted to hospital. The onset of the disease was started with dysarthria and weakness in upper extremities, and the patient was diagnosed as having MND by Huashan Hospital Affiliated to Fudan University in April 2014. Electromyogram (April, 2014, Huashan Hospital) showed motor neuron lesion-induced electromyographic changes, affecting the upper and lower extremities, rectus abdominis, tongue muscles and trapezius muscles, and anterior horn cell lesion in spinal cord was considered. The patient was treated with neurotrophic agent and oral protilatin (1#, bid), but the efficacy was poor. The patient was admitted to Wujin Hospital and received treatment with the new therapy from April 28, 2015 to May 19, 2015 and from November 30, 2015 to December 10, 2015. Upon admission to hospital, the patient had progressive limb muscle atrophy with weakness, difficulty in walking on flat ground and risk of falling over, and could not walk up stairs. The limbs had the sensation of muscle twitch and numbness, and vague speech, cough upon drinking water and eating, difficulty in breathing, and continuous need of non-invasive ventilators for facilitating breathing existed. In early 8 months after hospitalization, the patient had a weight loss of 20.5 kg. The patient also had tongue atrophy and fibrillation; interosseous muscle atrophy of two hands, muscle atrophy of extremities, handgrip strength of grade IV-, proximal muscle strength of both upper extremities of grade V-, muscle strength of both lower extremities of grade V-, positive Babinski sign, and hyperreflexia of tendon reflexes. The patient was further particularly diagnosed as having MND (amyotrophic lateral sclerosis). When the patient was admitted to hospital, he could not complete a pulmonary function test.

Specific therapeutic regimen: 500 ml of compound amino acid injection/day, intravenous infusion, once a day; 250 ml of 5% glucose and sodium chloride injection + 3.0 g of vitamin B₆ + 2.0 g of vitamin C, once a day, intravenous infusion.

After 5 days of continuous medication, the clinical symptoms such as shortness of breath and dysphagia were significantly improved. After 15 days of continuous medication, the dry mouth, weight loss, sensation of muscle twitch, numbness of hands, general muscle atrophy, weakness, cough upon drinking water, panting and other symptoms of the patients were obviously improved. The proximal muscle strength of the upper extremities and the muscle strength of the lower extremities were significantly increased, and the patient was able to walk freely. After continuing to receive the therapy for 1 week, the sensations of cough, panting, weakness, and blurred vision were ameliorated. No non-invasive ventilator was needed when discharged from hospital. The weight gain was 2 kg. The muscle strength of the lower extremities was increased from grade V- to grade V. The pulmonary function test conducted on December 08, 2015 showed moderate pulmonary dysfunction.

After the last discharge from hospital, the patient has not received the therapy any longer. The 3rd follow-up showed that the patient's condition was worse than that at the time of discharge from hospital, an invasive ventilator was needed to facilitate breathing (October, 2016) and intubation feeding was needed (October, 2016). This suggests that although this therapy can reverse the condition, if the treatment is discontinued for a long time, the condition will continue to develop.

### Detection of lymphocyte subgroups by PCR

| Examination Item | Examination time | | Reference range |
|---|---|---|---|
| | 12/01/2015 | 12/09/2015 | |
| Total T cell (%) | 80.01↑ | 74.70 | 61.10-77.0 |
| T-helper cell (%) | 47.12↑ | 45.15↑ | 25.80-41.60 |
| Suppressor T cell (%) | 30.41↑ | 30.28↑ | 18.10-29.60 |
| CD4/CD8 ratio | 1.55 | 1.36 | 0.71-2.78 |
| NK cell (%) | 8.21 | 9.53 | 8.10-35.60 |
| B lymphocyte (%) | 10.80 | 13.97 | 5.00-18.00 |

By comparing the detection results of lymphocyte subgroups before and after treatment, it can be seen that the immunologic function of the patient has also been improved.

### ALSFRS-R score

| | First admission to hospital | Last discharge from hospital | 1st follow-up | 2nd follow-up | 3rd follow-up |
|---|---|---|---|---|---|
| Time | 04/28/2015 | 12/10/2015 | 03/10/2016 | 07/02/2016 | 10/20/2016 |
| Score | 23 | 24 | 24 | 24 | 21 |

### Case 2:

Patient: Liu X, male, aged 52 years. The onset time was September, 2013, and the course of disease had continued for 24 months when the patient was admitted to hospital. The onset of the disease was started with impaired mobility of the fingers of the left hand, and the patient was diagnosed as having motor neuron disease by Huashan Hospital Affiliated to Fudan University on September, 2013. Electromyography (2016-09, Huashan Hospital) was performed. The patient was diagnosed as having MND (ALS) by Wujin Hospital, and admitted to Wujin Hospital and received treatment with the therapy from September 14, 2015 to September 30, 2015. Upon admission to hospital, the patient had occasional cough upon drinking water and eating, difficult speaking and vague speech, inability to look up, progressive limb weakness, obvious muscle wasting of the left hand and the upper extremities and progressively exacerbated muscle wasting of the left hand and the upper extremities, and could walking slowly on flat ground but with risk of falling over. The patient also had tongue atrophy and fibrillation; interosseous muscle atrophy of two hands, muscle atrophy of extremities, grip strength of left hand of grade I, proximal muscle strength of left upper extremity of grade III, grip strength of right hand of grade IV, proximal muscle strength of right upper extremity of grade IV, muscle strength of both lower extremities of grade IV, and positive Babinski sign.

Specific therapeutic regimen: 500 ml of compound amino acid injection/day, intravenous infusion, once a day; 250 ml of 5% glucose and sodium chloride injection + 3.0 g of vitamin B₆ + 2.0 g of vitamin C, once a day, intravenous infusion.

When the patient was admitted to hospital, he could only walk 200 meters. After 2 weeks of treatment, he could walk 3 kilometers independently. The weakness symptom was improved, and the muscle strength of the left hand and upper limbs was increased significantly. At the time of discharge from hospital, the grip strength of the left hand was increased from grade I to grade III, and the muscle strength of both lower limbs was increased from grade IV to grade V.

### Detection of lymphocyte subgroups by PCR

| Examination item | Examination time | | Reference range |
|---|---|---|---|
| | 09/15/2015 | 09/29/2015 | |
| Total T cell (%) | 74.36 | 71.74 | 61.10-77.0 |
| T-helper cell (%) | 48.71↑ | 39.35 | 25.80-41.60 |
| Suppressor T cell (%) | 20.57 | 22.98 | 18.10-29.60 |
| CD4/CD8 ratio | 2.37 | 1.71 | 0.71-2.78 |
| NK cell (%) | 4.34↓ | 11.85 | 8.10-35.60 |
| B lymphocyte (%) | 20.68↑ | 15.23 | 5.00-18.00 |

By comparing the detection results of lymphocyte subgroups before and after treatment, it can be seen that the immunologic function of the patient has also been improved.

### ALSFRS-R score

| | Admission to hospital | Discharge from hospital | Follow-up |
|---|---|---|---|
| Time | 09/14/2015 | 09/30/2015 | 08/5/2016 |
| Score | 38 | 39 | 39 |

### Case 3:

Patient: Hao XX, male, aged 60 years. The onset time was August, 2013, and the course of disease had continued for 27 months when the patient was admitted to hospital. The onset of the disease was started with weakness of the right arm. The patient was diagnosed as having MND (AMS) by the Third Hospital affiliated to Peking University (June, 2014), and the Guanganmen Hospital, the Chinese Academy of Chinese Medical Sciences (June 22, 2014). Electromyography (June 18, 2014, The First Affiliated Hospital affiliated to Peking University, 141515; the Third Hospital affiliated to Peking University), pulmonary function test, and cervical MRI (2014-06-10, Xuanwu Hospital affiliated to Capital Medical University, 00063326) were performed. The patient had received treatments with neuroprotective drugs, stem cell transplantation, traditional Chinese medicine, acupuncture and massage, and other therapies, but the efficacy was poor. The patient was admitted to Wujin Hospital and received treatment with the therapy from November 03, 2015 to November 18, 2015. Upon admission to hospital, the patient had progressive weakness of upper extremities, inability to look up and shrug the shoulders, and inability to compete a close-together motion by little fingers of both hands. The patient also had tongue fibrillation, muscle atrophy of both upper limbs, interosseous muscles, and shoulder muscles; muscle strength of bilateral deltoids of grade III-, muscle strength of biceps of grade III, slightly weakened grip strength, bilateral biceps reflex (+) , bilateral triceps reflex (+), and bilateral Hoffmann sign (+). Before hospitalization, the electromyogram showed neurogenic lesions of the right abductor pollicis brevis, bilateral biceps, and right sternocleidomastoid; and left abductor digiti minimi, and normal motor unit action potential duration and elevated amplitude upon slight contraction of T10 paraspinal muscle. Pulmonary function test (2015-05-15, the Third Hospital affiliated to Peking University, 16724) showed normal ventilation, and normal small airway function.

Specific therapeutic regimen: 500 ml of compound amino acid injection/day, intravenous infusion, once a day; 250 ml of 5% glucose and sodium chloride injection + 5.0 g of vitamin B₆ + 2.0 g of vitamin C, once a day, intravenous infusion.

After 10 consecutive days of treatment, the right little finger could complete the close-together motion, and the mobility of the right shoulder was increased. The atrophic muscles at the right elbow grew gradually.

### Detection of lymphocyte subgroups by PCR

| Examination item | Examination time | | Reference range |
|---|---|---|---|
| | 11/04/2015 | 11/17/2015 | |
| Total T cell (%) | 80.47↑ | 79.61↑ | 61.10-77.0 |
| T-helper cell (%) | 61.67↑ | 59.56↑ | 25.80-41.60 |
| Suppressor T cell (%) | 16.21↓ | 17.69↓ | 18.10-29.60 |
| CD4/CD8 ratio | 3.80↑ | 3.37↑ | 0.71-2.78 |
| NK cell (%) | 8.97 | 13.97 | 8.10-35.60 |
| B lymphocyte (%) | 8.83 | 5.75 | 5.00-18.00 |

The immunological function of the patient trends to be improved.

### ALSFRS-R score

| | Admission to hospital | Discharge from hospital | 1st follow-up | 2nd follow-up | 3rd follow-up |
|---|---|---|---|---|---|
| Time | 11/03/2015 | 11/18/2015 | 02/10/2016 | 05/12/2016 | 11/13/2016 |
| Score | 43 | 43 | 43 | 42 | 36 |

### Case 4:

Patient: Yu XX, male, aged 41 years. The onset time was March, 2007, and the course of disease had continued for 104 months when the patient was admitted to hospital. The onset of the disease was started with weakness of both legs. The patient was diagnosed as having MND by the Shanghai Hospital of Integrated Traditional Chinese and Western Medicine (March 29, 2007), the Changzhou Second People's Hospital affiliated to Nanjing Medical University (March 25, 2008), and the Zhongshan Hospital affiliated to Fudan University (April 10, 2008) and Nanjing Brain Hospital (May 21, 2015). Electromyography (March 30, 2007, Shanghai Hospital of Integrated Traditional Chinese and Western Medicine, 0707431; March 25, 2008, Changzhou Second People's Hospital, 14107), and craniocervical MRI (December 29, 2006, Changzhou Wujin People's Hospital, 08339) were performed. The patient had once received the treatment with oral butylphthalide (0.1, tid) and traditional Chinese medicine, but the efficacy was poor. The patient was diagnosed as having MND (ALS) by Wujin Hospital, and admitted to Wujin Hospital and received treatment with the therapy from Thursday, November 05, 2015 to Thursday, November 19, 2015. Upon admission to hospital, the patient had progressive weakness of limbs with the sensation of muscle twitch, especially at the left side of the body. The patient also had unsteady walking, difficulty in walk up stairs, numbness of hands, inability of the fingers to compete a close-together motion, and inability to raise both arms above the head, inability to look up and shrug the shoulders, vague speech, cough upon drinking water, and occasional panting. The patient also had tongue atrophy, interosseous muscle atrophy of two hands, muscle atrophy of extremities, and tongue muscle fibrillation; proximal muscle strength of both upper extremities of grade III, grip force of two hands of grade IV, muscle strength of lower extremities of grade IV; not high muscle tone; and weakened bilateral patellar reflex. Electromyogram (EMG) before admission to hospital showed fibrillation and positive sharp waves for the examined muscle of the upper and lower limbs, the rectus abdominis, and the sternocleidomastoid muscles; and broader and larger MUP or vast potential or poor waveform for some examined muscles upon light contraction; and reduced recruitment upon heavy contraction. NCV: reduced motor conduction velocity and CMAP amplitude of left deep branch of the ulnar nerve; normal conduction velocity and amplitude of remaining motor and sensory nerves; and normal F wave latency or non-elicited F wave of motor nerve. Electromyogram showed neurogenic lesions, affecting upper and lower extremities, rectus abdominis, trapezius muscles, and sternocleidomastoid muscle, and tongue muscles also had light chronic lesion. Lesions of the anterior horn cells in spinal cord and hypoglossal motor nerve damage were considered.

Specific therapeutic regimen: 500 ml of compound amino acid injection/day, intravenous infusion, once a day; 250 ml of 5% glucose and sodium chloride injection + 5.0 g of vitamin B₆ + 2.0 g of vitamin C, once a day, intravenous infusion.

After 2 weeks of treatment, the weakness symptoms were improved, the shoulders were stronger than before, and the arms could be raised above the head.

### Detection of lymphocyte subgroups by PCR

| Examination item | Examination time | | Reference range |
|---|---|---|---|
| | 11/06/2015 | 11/18/2015 | |
| Total T cell (%) | 62.79 | 72.08 | 61.10-77.0 |
| T-helper cell (%) | 37.91 | 43.62↑ | 25.80-41.60 |
| Suppressor T cell (%) | 23.88 | 26.91 | 18.10-29.60 |
| CD4/CD8 ratio | 1.59 | 1.62 | 0.71-2.78 |
| NK cell (%) | 16.33 | 18.81 | 8.10-35.60 |
| B lymphocyte (%) | 17.23 | 7.83 | 5.00-18.00 |

### ALSFRS-R score

| | Admission to hospital | Discharge from hospital | 1st follow-up | 2nd follow-up | 3rd follow-up |
|---|---|---|---|---|---|
| Time | 11/05/2015 | 11/19/2015 | 02/19/2016 | 05/21/2016 | 11/13/2016 |
| Score | 32 | 33 | 33 | 33 | 32 |

### Case 5:

Patient: Huang XX, male, aged 52 years. The onset time was March, 2014, and the course of disease had continued for 20 months when the patient was admitted to hospital. The onset of disease was started with weakness of two legs. The patient was diagnosed as having MND (progressive muscle atrophy) by Huashan Hospital Affiliated to Fudan University (August 14, 2014), Xuanwu Hospital, Capital Medical University (December 22, 2014), and the Third Hospital affiliated to Peking University. Electromyography (August 14, 2014, Huashan Hospital, H302091; December 26, 2014, Xuanwu Hospital, 50916), tissue biopsy [December 30, 2014, Xuanwu Hospital, left quadriceps, N12(626955)], CSF, and serum immune parameter analysis (August 15, 2014, Huashan Hospital, 20354), CSF capsid protein related antibodies (December 23, 2014, Xuanwu Hospital, 41602678), serum capsid protein related antibodies (August 19, 2014, Huashan Hospital, 1952), and blood and urine toxin analysis (December 26, 2014, 307 hospital affiliated to Academy of Military Medical Sciences), and other auxiliary examinations were performed. The patient had received treatments with co-enzyme Q10 (10 mg, p.o., tid), butylphthalide (0.2, p.o., bid), liraglutide (1#, p.o., bid), high-dose hormone shock and traditional Chinese medicine, but the efficacy was poor. The patient was admitted to Wujin Hospital and received treatment with the therapy from November 13, 2015 to November 29, 2015. Upon admission to hospital, the patient had progressive weakness of limbs, paroxysmal convulsions with pain, inability to shrug shoulders, complete loss of walking ability of double lower extremities, sensation of muscle twitch, fibrillation, but non-atrophy of tongue muscles, and even muscle atrophy of the shoulders and both lower extremities. The patient also had muscle strength of bilateral upper extremity of grade V-, slightly weakened grip strength, muscle strength of lower extremities of grade 0, no muscle tone, weakened muscle strength of of the waist, patellar reflex (-), and weakened deep sensations. Electromyogram (EMG) before admission to hospital showed fibrillation and positive sharp wave or fasculation potential for the examined muscle of the upper and lower limbs, the rectus abdominis, the trapezius muscles and the sternocleidomastoid muscles; broader and larger MUP or vast potential with/without polyphasic potential and increased irregular waves for some examined muscle upon light contraction; and reduced recruitment upon heavy contraction. NCV: reduced motor conduction velocity and CMAP amplitude of bilateral common peroneal nerve; normal conduction velocity and amplitude of remaining motor and sensory nerves; and normal F wave latency or non-elicited F wave of motor nerve. Electromyogram showed neurogenic lesion-induced electromyographic changess, affecting the muscles in upper and lower extremities, rectus abdominis, trapezius muscles, and sternocleidomastoid muscle. Lesions of the anterior horn cells in spinal cord were considered.

Specific therapeutic regimen: 500 ml of compound amino acid injection/day, intravenous infusion, once a day; 250 ml of 5% glucose and sodium chloride injection + 5.0 g of vitamin B₆ + 2.0 g of vitamin C, once a day, intravenous infusion.

After 10 days of treatment, the patient was able to stand on his own for 6 minutes. The muscle strength and muscle nutrition of both lower limbs were significantly improved compared with the case before treatment. The muscles of the foot contracted and the knee jerk reflex decreased. The muscle tone of both lower limbs was increased compared with that at the time of admission.

### Detection of lymphocyte subgroups by PCR

| Examination Item | Examination time | | Reference range |
|---|---|---|---|
| | 11/14/2015 | 11/27/2015 | |
| Total T cell (%) | 78.87↑ | 69.28 | 61.10-77.0 |
| T-helper cell (%) | 52.38↑ | 41.16 | 25.80-41.60 |
| Suppressor T cell (%) | 22.85 | 24.73 | 18.10-29.60 |
| CD4/CD8 ratio | 2.29 | 1.66 | 0.71-2.78 |
| NK cell (%) | 9.68 | 22.75 | 8.10-35.60 |
| B lymphocyte (%) | 9.04 | 7.06 | 5.00-18.00 |

By comparing the detection results of lymphocyte subgroups before and after treatment, it can be seen that the immunologic function of the patient has also been improved.

### ALSFRS-R score

| | Admission to hospital | Discharge from hospital | 1st follow-up | 2nd follow-up | 3rd follow-up |
|---|---|---|---|---|---|
| Time | 11/13/2015 | 11/29/2015 | 02/22/2016 | 06/04/2016 | 11/14/2016 |
| Score | 35 | 36 | 36 | 30 | 28 |

### Case 6:

Patient: Huang XX, male, aged 50 years. The onset time was April, 2014, and the course of disease had continued for 29 months when the patient was admitted to hospital. The onset of disease was started with weakness of the right leg. The patient was diagnosed as having motor neuron disease (possible lower motor neuron syndrome and spinal muscular atrophy) by the East Hospital affiliated to Tongji University (April 05, 2016), Shuguang Hospital affiliated to Shanghai University of Traditional Chinese Medicine (June 17, 2016), and Xinhua Hospital affiliated to School of Medicine, Shanghai Jiaotong University (April 22, 2016). Electromyography (April 05, 2016, East Hospital affiliated to Tongji University, 147591; April 22, 2016, Xinhua Hospital affiliated to School of Medicine, Shanghai Jiaotong University, 40621; June 17, 2016, Shuguang Hospital affiliated to Shanghai University of Traditional Chinese Medicine, 24172; and April 25, 2016, Xinhua Hospital affiliated to School of Medicine, Shanghai Jiaotong University, 40621), and craniocervical thoracolumbar MR (April 07, 2016, East Hospital affiliated to Tongji University, M13155250, M13155249, M13155247, M13155248) were performed. The patient had received treatment with many oral drugs (Ubidecarenone capsule, 2#, p.o., tid; vitamin E-10 capsule, 1#, p.o., tid; vitamin B₁ tablet, 1#, p.o., tid; and Mecobalamin tablet, 1#, p.o., tid) and traditional Chinese medicines, but the efficacy was undesirable. The patient was admitted to Wujin Hospital and received treatment with the therapy from September 29, 2016 to October 27, 2016. At the time of admission to hospital, the patient had progressive weakness of limbs with inflexible mobility, heavy lower extremities, sensation of occasional muscle twitch of two legs, and difficulty in waking up stairs with risk of fall over. The lower extremities could be lifted by 10 cm on bed. The patient also had muscle strength of left iliopsoas of grade V-, muscle strength of gluteus maximus of grade V-, and muscle strengths of bilateral quadriceps, tibialis anterior muscles, and posterior calf muscles of grade IV, slightly decreased muscle tone, gag reflex (+), bilateral biceps reflex (++), bilateral patellar reflex (+), and ankle reflex (+). The electromyogram (Shuguang Hospital affiliated to Shanghai University of Traditional Chinese Medicine) showed neurogenic disease affecting the examined muscle of the limbs, the rectus abdominis, and the paraspinal muscle that all had denervation changes, and trapezius muscles that had extended insertion potential and vast potential. MND was considered.

Specific therapeutic regimen: 250 ml of compound amino acid injection + 250 ml of 0.9% sodium chloride injection + 5.0 g of vitamin B₆, intravenous infusion, once a day.

After 4 weeks of hospitalization, the patient's sensation of weakness in the lower extremities was relieved, and the lower extremities could be lifted by 30 cm on the bed (a 20 cm increase compared with that at the time of admission to hospital) upon discharge from hospital.

### Detection of lymphocyte subgroups by PCR

| Examination item | Examination time | | | Reference range |
|---|---|---|---|---|
| | 10/03/2016 | 10/11/2016 | 10/27/2016 | |
| Total T cell (%) | 81.80↑ | 80.60↑ | 82.47↑ | 61.10-77.0 |
| T-helper cell (%) | 39.07 | 42.48↑ | 41.27 | 25.80-41.60 |
| Suppressor T cell (%) | 35.51↑ | 32.06↑ | 35.00↑ | 18.10-29.60 |
| CD4/CD8 ratio | 1.10 | 1.33 | 1.18 | 0.71-2.78 |
| NK cell (%) | 8.16 | 5.85↓ | 7.61↓ | 8.10-35.60 |
| B lymphocyte (%) | 8.68 | 9.99 | 8.58 | 5.00-18.00 |

### Determination of blood myoglobin

| Examination item | Examination time | | | Reference range |
|---|---|---|---|---|
| | 09/30/2016 | 10/10/2016 | 10/26/2016 | |
| Myoglobin (ng/ml) | 107.10↑ | 222.30↑ | 145.20↑ | 28.00-72.00 |

### ALSFRS-R score

| | Admission to hospital | Discharge from hospital |
|---|---|---|
| Time | 09/29/2016 | 10/27/2016 |
| Score | 40 | 41 |

### Case 7:

Patient: Zhu XX, male, aged 50 years. The onset time was September, 2015, and the course of disease had continued for 13 months when the patient was admitted to hospital. The onset of disease was started with weakness of the left leg and impaired mobility of the left toes. The patient was diagnosed as having MND by the First Hospital affiliated to Suzhou University (September 24, 2015) and Zhejiang Hospital of Traditional Chinese Medicine (March 01, 2016). Electromyography (September 24, 2015, the First Hospital affiliated to Suzhou University, 9755; March 01, 2016, Zhejiang Hospital of Traditional Chinese Medicine, 160367-05705; and May 30, 2016, the First Hospital affiliated to Suzhou University, 11332) was performed. The patient had received treatment with many drugs [Mecobalamin, 0.5 mg, p.o., tid; fursultiamine, 25 mg, p.o., tid; prednisone, 20 mg, p.o., qd (minus 1 tablet every 5 days); Ginkgolides, 1#, p.o., tid], but the efficacy was undesirable. The patient was diagnosed as having MND (ALS) by Wujin Hospital, and admitted to Wujin Hospital and received treatment with the therapy from September 28, 2016 to October 28, 2016. At the time of admission to hospital, the patient had weakness of extremities with obvious sensation of muscle twitch, especially the lower extremities, difficulty in walking with risk of fall over, forced hyperextension and limited mobility of left toes. The patient also had muscle atrophy of bilateral biceps, bilateral quadriceps, tibialis anterior muscles, and posterior calf muscles; muscle strength of iliopsoas of grade IV, muscle strength of bilateral quadriceps of grade IV, muscle strength of left posterior calf muscles of grade III, muscle strength of right posterior calf muscles of grade IV, and muscle strength of bilateral tibialis anterior muscles of grade III; plantar flexion and dorsiflexion weakness of right foot; low muscle tone of the limbs, and elevated muscle tone of the left toes; and disappeared biceps reflex. The sister of the patient was afflicted with the same disease. Before admission to hospital, the electromyogram showed 1. reduced amplitude of potentials evoked by motor nerve conduction of right common peroneal nerve; 2. prolonged distal latency of and reduced amplitude of potentials evoked by motor nerve conduction of bilateral nervus tibialis; 3. small motor response of motor nerve conduction of left common peroneal nerve ; 4. fasculation potential upon resting of right abductor digiti minimi, which appeared to have MUP of normal level upon light contraction, and interference pattern upon strong contraction; 5. neurogenic lesion-induced electromyographic changes of left quadriceps, right tibialis anterior muscle, and left tibialis anterior muscle; and 6. presence of spontaneous potential spike upon resting of right quadriceps, and presence of partially broad and large MUPs upon light contraction.

Specific therapeutic regimen: 250 ml of compound amino acid injection + 250 ml of 5% glucose and sodium chloride injection + 5.0 g of vitamin B₆ + 2.0 g of vitamin C + 4U insulin + 5 ml of 10% KCl, intravenous infusion, once a day.

After 30 days of regular treatment with this therapy, the weakness symptom of the limbs was relieved, and the forced hyperextension of the left toes disappeared. The weight gain was 1 kg before that at the time of admission to hospital. The elevated muscle tone of the left foot was obviously alleviated, and the mobility was enhanced. When discharged from hospital, the electromyogram performed for reexamination showed 1. fibrillation and positive sharp wave for examined muscles in the upper and lower extremities; 2. presence of broad and large MUPs of bilateral first dorsal interosseous muscle, right tibialis anterior muscle, and flexor carpi radiali upon light contraction; 3. presence of broad and large MUPs of bilateral peroneal medialis and right vastus medialis; 4. poor MUP waveform of right vastus medialis and left Tibialis anterior muscle; 5. prolonged distal latency of bilateral median nerve motor conduction; 6. reduced motor conduction CMAP amplitude of left median nerve, bilateral common peroneal nerve, and left nervus tibialis; 7. reduced left common peroneal nerve motor conduction velocity; and 8. reduced sensory conduction SANP amplitude of right nervus ulnaris. As shown by comparison of the electromyograms before and after hospitalization, a corresponding motor potential wave could be determined for the left common peroneal nerve that previously had only small motor response, and most of the affected nerve conduction amplitudes were elevated.

### Detection of lymphocyte subgroups by PCR

| Examination item | Examination time | | | Reference range |
|---|---|---|---|---|
| | 09/30/2016 | 10/10/2016 | 10/27/2016 | |
| Total T cell (%) | 76.24 | 74.58 | 80.23↑ | 61.10-77.0 |
| T-helper cell (%) | 40.96 | 43.79↑ | 48.91↓ | 25.80-41.60 |
| Suppressor T cell (%) | 20.82 | 20.11 | 21.35 | 18.10-29.60 |
| CD4/CD8 ratio | 1.97 | 2.18 | 2.29 | 0.71-2.78 |
| NK cell (%) | 16.00 | 18.56 | 11.31 | 8.10-35.60 |
| B lymphocyte (%) | 5.34 | 5.47 | 6.51 | 5.00-18.00 |

### Determination of blood myoglobin

| Examination item | Examination time | | | Reference range |
|---|---|---|---|---|
| | 09/29/2016 | 10/09/2016 | 10/26/2016 | |
| Myoglobin (ng/ml) | 78.15↑ | 59.90 | 100.90↑ | 28.00~72.00 |

### ALSFRS-R score

| | Admission to hospital | Discharge from hospital |
|---|---|---|
| Time | 09/28/2016 | 10/28/2016 |
| Score | 33 | 34 |

### Case 8:

Patient: Xu XX, male, aged 66 years. The onset time was May, 2015, and the course of disease had continued for 17 months when the patient was admitted to hospital. The onset of the disease was started with dysarthria, and the patient was diagnosed as having motor neuron disease by Huashan Hospital Affiliated to Fudan University (March 28, 2016). Electromyography (February 16, 2016, Jiangsu People's Hospital, C20160181; March 28, 2016, Huashan Hospital Affiliated to Fudan University, 58927), craniocervical and lumbosacral MRI (June 09, 2015, Gulou Hospital, cranial MRI+MRA; September 2015, Zhongda Hospital affiliated to Southeast University, cranial MRI+MRA, neck, chest, lumbosacral, 964151XGT), and pulmonary function test (February 04, 2016, Jiangsu Hospital of Traditional Chinese Medicine, KSXUGT16020402) were performed. The patient had received treatment with liraglutide, but the efficacy was poor. The patient was diagnosed as having MND (ALS) by Wujin Hospital, and admitted to Wujin Hospital and received treatment with the therapy from October 17, 2016 to November 13, 2016. At the time of admission to hospital, the patient had vague speech with drooling, dysphagia, cough upon drinking water and eating, weakness of the limbs, particularly the left limbs, sensation of muscle twitch of the left lower extremities, unsteady walking, and the above symptoms are progressively aggravated. The patient also had obvious tongue atrophy and fibrillation; grip strength of the left hand of grade IV, proximal muscle strength of the left upper extremities of grade IV, muscle strength of the bilateral tibialis anterior muscle of grade IV, plantar flexion and dorsiflexion weakness of feet, slightly reduced muscle tone of limbs, and disappeared biceps reflex. Before admission to hospital, the electromyogram (EMG) showed fibrillation of left tongue muscles, (the right tongue muscles were unexamined); presence of positive sharp waves for the left first dorsal interosseous muscle, flexor carpi radiali, tongue muscles, and right flexor carpi radiali; fasciculation of the right sternocleidomastoid muscle; extended insertion potential of some examined muscles in the upper and lower extremities, broad and large MUPs with/without polyphasic potential and increased irregular waves upon light contraction; and reduced recruitment upon impulse contraction. NCV: normal conduction velocity and amplitude of the examined motor and sensory nerves. Normal value for F wave latency of motor nerve. Blink: normal R1 and R2 latency ranges for orbicularis oculi muscle recorded upon stimulation of bilateral incisura supraorbitalis. Electromyogram showed neurogenic lesion-induced electromyographic changes, affecting mainly the tongue muscles, and also some muscles in the upper extremities and the sternocleidomastoid muscle to a small extent.

Specific therapeutic regimen: 300 ml of compound amino acid injection + 250 ml of 0.9% sodium chloride injection + 3.0 g of vitamin B₆ + 2.0 g of vitamin C, intravenous infusion, once a day.

After 30 days of regular treatment with this therapy, the weakness of limbs of the patient was significantly improved, and the mobility of the left lower extremity was more flexible, and the drooling decreased. At the time of discharge from hospital, the electromyogram (EMG) performed for reexamination showed fibrillation of bilateral tongue muscles, positive sharp waves for right tibialis anterior muscle and bilateral tongue muscles, broad MUPs or partially broad and large MUPs for some examined muscles upon light contraction, and reduced recruitment upon heavy contraction. NUV: normal conduction velocity and amplitude of the examined motor and sensory nerves. Electromyogram showed neurogenic lesion-induced electromyographic changes, affecting mainly the tongue muscles, and also some muscles in the upper and lower extremities and the musculus trapezius to a small extent. Lesions of the anterior horn cells in spinal cord and hypoglossal motor nerve damage were considered.

### Detection of lymphocyte subgroups by PCR

| Examination item | Examination time | | Reference range |
|---|---|---|---|
| | 10/19/2016 | Discharge from hospital | |
| Total T cell (%) | 49.90↓ | Not available | 61.10-77.0 |
| T-helper cell (%) | 42.04↑ | | 25.80-41.60 |
| Suppressor T cell (%) | 6.66↓ | | 18.10-29.60 |
| CD4/CD8 ratio | 6.31↑ | | 0.71-2.78 |
| NK cell (%) | 33.87 | | 8.10-35.60 |
| B lymphocyte (%) | 13.28 | | 5.00-18.00 |

### Determination of blood myoglobin

| Examination Item | Examination time | | Reference range |
|---|---|---|---|
| | 10/18/2016 | 11/07/2016 | |
| Myoglobin (ng/ml) | 34.06 | 56.7 | 28.00-72.00 |

### ALSFRS-R score

| | Admission to hospital | Discharge from hospital |
|---|---|---|
| Time | 10/17/2016 | 11/13/2016 |
| Score | 34 | 34 |

### Case 9:

Patient: Wang XX, male, aged 53 years. The onset time was September, 2015, and the course of disease had continued for 13 months when the patient was admitted to hospital. The onset of disease was started with weakness of right fingers. The patient was diagnosed as having MND by Zhongda Hospital affiliated to Southeast University (October 11, 2016). Electromyography (October 10, 2016, Zhongda Hospital affiliated to Southeast University, 000875) and craniocervical MRI (February 2, 2016, Tianchang Hospital of Chinese Traditional Medicine) were performed. The patient had received treatment with oral liraglutide (1#, p.o., bid), circulation improving agent, and neurotrophic agent, but the efficacy was poor, and the condition has developed rapidly. The patient was diagnosed as having MND (ALS) by Wujin Hospital, and admitted to Wujin Hospital and received treatment with the therapy from October 12, 2016 to November 08, 2016. At the time of admission to hospital, the patient had progressive weakness of limbs, especially the left limbs, slow walking with the risk of fall over, inability to comb the hair and lift heavy stuffs by the upper extremities, inability to raise the shoulders with the sensation of muscle twitch, and vague speech. The patient also had interosseous muscle atrophy and deformity of both hands, muscle atrophy of bilateral biceps, grip strength of right hand of grade I-II, grip strength of left hand of grade III, proximal muscle strength of left upper extremities of grade I, proximal muscle strength of right upper extremities of grade IV, muscle strength of iliopsoas of grade IV, muscle strength of bilateral posterior calf muscles of grade IV, muscle strength of bilateral tibialis anterior muscles of grade IV, plantar flexion and dorsiflexion slight weakness of feet, low muscle tone of limbs, and disappeared biceps reflex. Before admission to hospital, the electromyogram (EMG) (October 10, 2016, Zhongda Hospital affiliated to Southeast University) showed presence of a large number of spontaneous potentials of right deltoid and right tibialis anterior muscle when in a quiescent state, widened motor unit potential duration upon small-force contraction, simple pattern of recruitment upon strong contraction; widened motor unit potential duration upon light contraction of left sternocleidomastoid muscle, and reduced recruitment upon strong contraction; and presence of a large number of spontaneous potentials when right T11, T10 were in a quiescent state. NCV: 1. high normal distal motor latency, normal conduction velocity, reduced CMAP amplitude, and non-elicited F wave; and normal sensory conduction velocity and normal SNAP amplitude, of right median nerve. 2. prolonged distal motor latency, normal conduction velocity, reduced CMAP amplitude, and non-elicited F wave; and sensory normal conduction velocity and normal SNAP amplitude, of right nervus ulnaris. Generalized neurogenic lesion was considered.

Specific therapeutic regimen: 250 ml of compound amino acid injection + 250 ml of 0.9% sodium chloride injection + 3.0 g of vitamin B₆ + 2.0 g of vitamin C + 10 ml of 10% KCl, intravenous infusion, once a day.

After more than 20 days of regular treatment with the therapy, the patient had improved weakness in the lower limbs and was able to walk more actively than when he was admitted to the hospital. The improvement of the remaining symptoms was not obvious, but the progression of the disease was significantly reduced and the condition was controlled to some extent. Electromyogram (EMG) (November 04, 2016) at the time of discharge from hospital showed fibrillation and positive sharp wave for the examined muscle of the upper and lower limbs and the tongue muscles, broad and large MUPs or partially broad and large MUPs for some examined muscles upon light contraction; and reduced recruitment upon heavy contraction. NCV: reduced conduction CMAP amplitude of right median nerve and nervus ulnaris; and normal conduction velocity and amplitude of remaining motor and sensory nerves. Electromyogram showed neurogenic lesion-induced electromyographic changes, affecting mainly the muscles in upper and lower extremities and the tongue muscles, and also trapezius muscles to a small extent. Lesions of the anterior horn cells in spinal cord and hypoglossal motor nerve damage were considered.

### Detection of lymphocyte subgroups by PCR

| Examination item | Examination time | | Reference range |
|---|---|---|---|
| | 10/14/2016 | Discharge from hospital | |
| Total T cell (%) | 61.57 | Not available | 61.10-77.0 |
| T-helper cell (%) | 42.80↑ | | 25.80-41.60 |
| Suppressor T cell (%) | 16.52↓ | | 18.10-29.60 |
| CD4/CD8 ratio | 2.59 | | 0.71-2.78 |
| NK cell (%) | 15.63 | | 8.10-35.60 |
| B lymphocyte (%) | 19.69↑ | | 5.00-18.00 |

### Determination of blood myoglobin

| Examination item | Examination time | | Reference range |
|---|---|---|---|
| | 10/13/2016 | Discharge from hospital | |
| Myoglobin (ng/ml) | 50.08 | Not available | 28.00-72.00 |

### ALSFRS-R score

| | Recollection evaluation | | | Admission to hospital | Discharge from hospital |
|---|---|---|---|---|---|
| Time | 09/2015 | 03/2016 | 09/2016 | 10/13/2016 | 11/08/2016 |
| Score | 47 | 36 | 28 | 27 | 27 |

### Case 10:

Patient: Tian X, female, aged 51 years. The onset time was October, 2012, and the course of disease had continued for 39 months when the patient was admitted to hospital. The onset of disease was started with weakness of lower extremities. The patient was diagnosed as having undefined monoclonal gammopathy and myopathy by Xuanwu Hospital affiliated to Capital Medical University (April 16, 2014, Medical Record No: 605437), and the Third Hospital affiliated to Peking University (November 02, 2014, Hospital Admission No: 4770669). A number of related examinations were performed during medical treatment at various medical institutions, including cervical and lumbar MRI (February 25, 2014, Air Force General Hospital); serum protein electrophoresis, immunoglobulin quantification, immunofixation electrophoresis, Bence-Jone's protein urine test, thigh MRI, muscle biopsy of left quadriceps, electromyography, amino acid and carnitine profile test, urine organic acid profile analysis, blood and urine toxin analysis (March 26, 2014 to April 10, 2014, during hospitalization in Xuanwu Hospital); PET/ CT (April 21, 2014, PLA General Hospital); immunoglobulin fixation electrophoresis, electromyography, muscle biopsy of right biceps femoris, and bone marrow biopsy; determination of TB lymphocyte subgroups, CA125, qualitative analysis of Bence-Jone's protein, carcinomacmbryonic antigen, rheumatism-related three criteria (ASO RF CRP), immune-related several criteria, cerebrospinal fluid routine test, tumor-specific growth factors, systemic + local bone imaging, leukemia/lymphoma immunophenotyping (from December 02, 2014 to December 06, 2014, during hospitalization in the Third Hospital affiliated to Peking University); gene detection report for single-gene genetic disease (March 30, 2015, Shenzhen Huada Clinical Laboratory Center); and KAP + LAM (May 22, 2015, Peking Union Medical College Hospital). The patient had received treatments with hormone, B vitamins, traditional Chinese medicines, stem cell transplantation, and neurotrophic agents, but the efficacy was poor. The patient was diagnosed as having MND (progressive amyotrophy) by Wujin Hospital, and admitted to Wujin Hospital and received treatment with the therapy from January 18, 2016 to January 30, 2016. At the time of admission to hospital, the patient had progressively exacerbated weakness of the limbs with obvious muscle atrophy, inability of the lower extremities to walk independently, sensation of occasional muscle twitch of the thigh, weakened strength in cough expectoration, inability to look up and shrug shoulder, difficulty in breathing, and need of non-invasive ventilators to facilitate breathing intermittently. The patient also had atrophy of bilateral biceps, handgrip strength of grade V, and proximal muscle strength of upper extremities of grade I; no swelling of lower extremities, and obvious muscle atrophy of bilateral quadriceps femoris; muscle strength of iliopsoas of grade II, muscle strength of bilateral quadriceps of grade 0, and muscle strength of bilateral posterior calf muscles of grade II; muscle strength of bilateral tibialis anterior muscles of grade II; muscle strength of left dorsal extensor hallucis muscle of grade II, muscle strength of right dorsal extensor hallucis muscle of grade II, and low muscle tone; and existence of pain and temperature sensation, and non-elicited tendon reflex. Two electromyograms before admission to hospital showed myogenic lesion. Muscle biopsy of left quadriceps (April 01, 2014, Xuanwu Hospital affiliated to Capital Medical University) showed neurogenic lesion.

Specific therapeutic regimen: 500 ml of compound amino acid injection, intravenous infusion, once a day; 500 ml of 0.9% sodium chloride injection + 5.0 g of vitamin B₆, intravenous infusion, once a day. After regular treatment with this therapy, the weakness symptom was improved slightly, the muscle strength of the neck and limbs was increased slightly, the atrophy of bilateral trapezius muscles and gluteus maximus was obviously improved, and the muscles were fuller than those upon admission to hospital. After being admitted to hospital and receiving the treatment for 10 days, the patient could stand up and walk (shuffle) about 5 meters with the support of some other person. The muscle strength of bilateral quadriceps was increased from grade 0 to grade I.

### Detection of lymphocyte subgroups by PCR

| Examination item | Examination time | | Reference range |
|---|---|---|---|
| | 01/19/2016 | 01/29/2016 | |
| Total T cell (%) | 49.60↓ | 62.68 | 61.10-77.0 |
| T-helper cell (%) | 26.49 | 39.38 | 25.80-41.60 |
| Suppressor T cell (%) | 21.16 | 21.48 | 18.10-29.60 |
| CD4/CD8 ratio | 1.25 | 1.79 | 0.71-2.78 |
| NK cell (%) | 29.20 | 18.48 | 8.10-35.60 |
| B lymphocyte (%) | 19.55↑ | 18.04↑ | 5.00-18.00 |

### ALSFRS-R score

| | Admission to hospital | Discharge from hospital | 1st follow-up | 2nd follow-up | 3rd follow-up |
|---|---|---|---|---|---|
| Time | 01/18/2016 | 01/30/2016 | 04/30/2016 | 08/02/2016 | 11/10/2016 |
| Score | 33 | 34 | 34 | 33 | 32 |

### Case 11:

Patient: Tao XX, male, aged 64 years. The onset time was March, 2014, and the course of disease had continued for 31 months when the patient was admitted to hospital. The onset of the disease was started with dysarthria and difficult swallowing, and the patient was diagnosed as having motor neuron disease (amyotrophic lateral sclerosis) by Huashan Hospital Affiliated to Fudan University (June 26, 2015), Peking Union Medical College Hospital (April 25, 2016), and Zhejiang Taizhou Hospital (June 18, 2015). Electromyography (June 18, 2015, Zhejiang Taizhou Hospital) was performed. The patient had received treatments with oral liraglutide (1#, p.o., bid, received continuous oral administration since April), and traditional Chinese medicine, but the efficacy was poor. The patient was admitted to Wujin Hospital and received treatment with the therapy from October 18, 2016 to December 05, 2016. At the time of admission to hospital, the patient had progressive vague speech, weakness of the limbs, and inability to shrug the shoulders and look up, accompanied by obvious drooling and dysphagia. The body loss was about 10 kg. The patient also had muscular atrophy of limbs and tongue muscle, proximal muscle strength of the left upper extremity of grade IV, muscle strength of bilateral tibialis anterior muscle of grade IV; plantar flexion and dorsiflexion weakness; slightly low muscle tone of limbs; and biceps tendon reflex (-). The electromyogram before admission to hospital showed generalized neurogenic lesion.

Specific therapeutic regimen: 300 ml of compound amino acid injection + 3.0 g of vitamin B₆ + 2.0 g of vitamin C + 250 ml of 0.9% sodium chloride injection, intravenous injection, once a day; 10 mg of racanisodamine tablet (oral administration). After regular treatment with the therapy, the drooling symptom was significantly reduced, the dysphagia was significantly improved compared with that at the time of admission to hospital, and the general weakness was relieved. At the time of discharge from hospital, the electromyogram (EMG) performed for reexamination showed fibrillation and positive sharp wave for some examined muscles in the upper extremities and for trapezius muscles, broad and large MUPs or partially broad and large MUPs for some muscles upon light contraction, and reduced recruitment upon impulse contraction. NCV: reduced motor conduction CMAP amplitude of right median nerve and nervus ulnaris; and normal conduction velocity and amplitude of remaining motor and sensory nerves. Electromyogram showed neurogenic lesion-induced electromyographic changes, affecting mainly the upper extremities, trapezius muscles, and sternocleidomastoid muscles, and also muscles in the lower extremities to a small extent. Lesions of the anterior horn cells in spinal cord were considered.

### Detection of lymphocyte subgroups by PCR

| Examination item | Examination time | | Reference range |
|---|---|---|---|
| | 10/20/2016 | 11/18/2016 | |
| Total T cell (%) | 64.29 | 64.34 | 61.10-77.0 |
| T-helper cell (%) | 45.57↑ | 43.26↑ | 25.80-41.60 |
| Suppressor T cell (%) | 17.51↓ | 19.82 | 18.10-29.60 |
| CD4/CD8 ratio | 2.60 | 2.18 | 0.71-2.78 |
| NK cell (%) | 23.62 | 27.25 | 8.10-35.60 |
| B lymphocyte (%) | 10.68 | 6.58 | 5.00-18.00 |

### Determination of blood myoglobin

| Examination item | Examination time | | Reference range |
|---|---|---|---|
| | 10/19/2016 | 11/17/2016 | |
| Myoglobin (ng/ml) | 42.79 | 95.79↑ | 28.00-72.00 |

### ALSFRS-R score

| | Admission to hospital | Discharge from hospital |
|---|---|---|
| Time | 10/18/2016 | 12/05/2016 |
| Score | 36 | 37 |

### Case 12:

Patient: Zhu XX, male, aged 77 years. The onset time was November, 2015, and the course of disease had continued for 8 months when the patient was admitted to hospital. The onset of disease was stared with dysarthria and less flexible tongue mobility. The patient was diagnosed as having motor neuron disease by the Changzhou Second People's Hospital affiliated to Nanjing Medical University (May, 2016). Electromyography (May, 2016, Changzhou Second People's Hospital; June 06, 2016, Renji Hospital affiliated to School of Medicine, Shanghai Jiaotong University, 2042) and Craniocervical MRI (May, 2016, Changzhou Second People's Hospital) were performed. The patient had received treatment with oral liraglutide (1#, p.o., bid), vitamin B₁ (1#, p.o., tid), and vitamin E (1#, p.o., tid), but the efficacy was poor. The patient was diagnosed as having MND (progressive bulbar palsy) by Wujin Hospital, and admitted to Wujin Hospital and received treatment with the therapy from July 28, 2016 to August 25, 2016. At the time of admission to hospital, the patient had vague speech with obvious drooling. The patient had numbness in the left extremities and shuffled walking and impaired mobility after the cerebral infarction in 2011. The patient also had atrophy and fibrillation of tongue muscles, muscle strength of left posterior calf muscles of grade IV, slight low muscle tone of the limbs, and retarded gag reflex. The electromyogram (EMG) before admission to hospital showed, for a small part of muscles examined, different degrees of positive sharp waves and fibrillation potential in a relaxed state, and reduced motor unit potential upon heavy contraction. CV: reduced MCV and SCV amplitudes of bilateral peroneal nerves, and normal SCV and MCV of the remaining nerves examined. RNS: obvious characteristic changes recorded upon stimulation of left and right facial nerves, right nervus ulnaris, right peroneal nerve, orbicularis oculi muscle, abductor digiti minimi, and extensor digitorum brevis.

Specific therapeutic regimen: 250 ml of compound amino acid injection, intravenous infusion, once a day; 250 ml of 0.9% sodium chloride injection + 3.0 g of vitamin B₆ + 5 ml of 10% KCl, intravenous infusion, once a day. After regular treatment with the therapy, the drooling symptom was significantly reduced, and the weakness of the limbs was improved.

### Detection of lymphocyte subgroups by PCR

| Examination item | Examination time | | Reference range |
|---|---|---|---|
| | 07/28/2016 | Discharge from hospital | |
| Total T cell (%) | 68.09 | Not available | 61.10-77.0 |
| T-helper cell (%) | 39.68 | | 25.80-41.60 |
| Suppressor T cell (%) | 18.07↓ | | 18.10-29.60 |
| CD4/CD8 ratio | 2.20 | | 0.71-2.78 |
| NK cell (%) | 18.42 | | 8.10-35.60 |
| B lymphocyte (%) | 12.53 | | 5.00-18.00 |

### ALSFRS-R score

| | Admission to hospital | Discharge from hospital | 1st follow-up |
|---|---|---|---|
| Time | 07/28/2016 | 08/25/2016 | 11/14/2016 |
| Score | 27 | 28 | 28 |

## Claims

1. A composition for use in a method for treating motor neuron diseases (MNDs) wherein the MNDs are amyotrophic lateral sclerosis, spinal muscular atrophy, primary lateral sclerosis, or progressive bulbar palsy, said composition comprising, in each unit, substances in amounts meeting the following proportional relationship, wherein "unit" refers to the daily dosage administered to a patient:
0.3 to 8 g of L-ornithine or an L-ornithine salt with an L-ornithine content equivalent to 0.3 to 8 g, 0.5 to 5 g of aspartic acid, and 2 to 20 g of vitamin B₆;
preferably 0.325 to 8 g of L-ornithine or an L-ornithine salt with an L-ornithine content equivalent to 0.325 to 8 g, 0.625 to 5 g of aspartic acid, and 3 to 20 g of vitamin B₆;
further preferably 0.5 to 8 g of L-ornithine or an L-ornithine salt with an L-ornithine content equivalent to 0.5 to 8 g, 1 to 5 g of aspartic acid, and 6 to 20 g of vitamin B₆;
or
further preferably 0.325 to 0.65 g of L-ornithine or an L-ornithine salt with an L-ornithine content equivalent to 0.325 to 0.65 g, 0.625 to 1.25 g of aspartic acid, and 3 to 10 g of vitamin B₆.

2. The composition for use according to claim 1, further comprising, in each unit, one or more of arginine, isoleucine, leucine, lysine, methionine, phenylalanine, threonine, tryptophan, valine, histidine, glycine, alanine, proline, asparagine, cysteine, glutamic acid, serine, tyrosine, vitamin B₁, vitamin B₂, vitamin B₃, pantothenic acid, biotin, folic acid, vitamin B₁₂, vitamin C, and KCl, wherein
the amino acids are optionally replaced by various soluble salts or derivatives thereof;
wherein the lysine is replaced by lysine acetate, the cysteine is replaced by N-acetyl-L-cysteine, and the tyrosine is replaced by N-acetyl-L-tyrosine, wherein the any one or more substances are used in amounts meeting the following proportional relationship:
the amino acids being used in amounts meeting the proportional relationship of 2.0-10 g of arginine, 2.0-10 g of isoleucine, 3.0-15 g of leucine, lysine acetate with a lysine content equivalent to 1.5-10 g, 0.2-3 g of methionine, 0.3-3 g of phenylalanine, 1.0-10 g of threonine, 0.3-3 g of tryptophan, 2.5-15 g of valine, 1.0-8 g of histidine, 1.5-8 g of glycine, 2.0-10 g of alanine, 1.5-8 g of proline, 0.1-3 g of asparagine, N-acetyl-L-cysteine with a cysteine content equivalent to 0.1-3 g, 1.0-10 g of glutamic acid, 0.5-5 g of serine, and N-acetyl-L-tyrosine with a tyrosine content equivalent to 0.1-3 g;
the vitamins being used in amounts meeting the proportional relationship of 1.0-4.0 mg of vitamin B₁, 1.0-4.0 mg of vitamin B₂, 10-40 mg of vitamin B₃, 3.0-10 mg of pantothenic acid, 0.1-0.4 mg of biotin, 0.1-0.8 mg of folic acid, 2.0-12 µg of vitamin B₁₂, and 1.0-6.0 g of vitamin C; and
KCl being used in an amount of 5-10 ml of 10% KCl.

3. The composition for use according to claim 2, wherein the amino acids being used in amounts meeting the proportional relationship of 2.2-4.4 g of arginine, 2.2-4.4 g of isoleucine, 3.4-6.8 g of leucine, lysine acetate with a lysine content equivalent to 1.8775-3.755 g, 0.3-0.6 g of methionine, 0.4-0.8 g of phenylalanine, 1.15-2.3 g of threonine, 0.375-0.75 g of tryptophan, 2.65-5.3 g of valine, 1.175-2.35 g of histidine, 1.575-3.15 g of glycine, 2.075-4.15 g of alanine, 1.775-3.55 g of proline, 0.1375-0.275 g of asparagine, N-acetyl-L-cysteine with a cysteine content equivalent to 0.15-0.3 g, 1.425-2.85 g of glutamic acid, 0.925-1.85 g of serine, and N-acetyl-L-tyrosine with a tyrosine content equivalent to 0.175-0.35 g;
the vitamins being used in amounts meeting the proportional relationship of 1.0-4.0 mg of vitamin B₁, 1.0-4.0 mg of vitamin B₂, 10-40 mg of vitamin B₃, 3.0-10 mg of pantothenic acid, 0.1-0.4 mg of biotin, 0.1-0.8 mg of folic acid, 2.0-12 µg of vitamin B₁₂, and 1.0-6.0 g of vitamin C; and
KCl being used in an amount of 5-10 ml of 10% KCl; or
the amino acids being used in amounts meeting the proportional relationship of 3.0-10 g of arginine, 3.0-10 g of isoleucine, 5.0-15 g of leucine, lysine acetate with a lysine content equivalent to 3.0-10 g, 0.5-3 g of methionine, 0.5-3 g of phenylalanine, 3.0-10 g of threonine, 0.5-3.0 g of tryptophan, 5.0-15 g of valine, 3.0-8.0 g of histidine, 3.0-8.0 g of glycine, 3.0-10 g of alanine, 3.0-8.0 g of proline, 0.1-3.0 g of asparagine, N-acetyl-L-cysteine with a cysteine content equivalent to 0.1-3.0 g, 3.0-10 g of glutamic acid, 0.5-5.0 g of serine, and N-acetyl-L-tyrosine with a tyrosine content equivalent to 0.1-3 g;
the vitamins being used in amounts meeting the proportional relationship of 2.0-4.0 mg of vitamin B₁, 2.0-4.0 mg of vitamin B₂, 20-40 mg of vitamin B₃, 6.0-10 mg of pantothenic acid, 0.2-0.4 mg of biotin, 0.2-0.8 mg of folic acid, 4.0-12 µg of vitamin B₁₂, and 2.0-6.0 g of vitamin C.

4. The composition for use according to any of claims 2-3, comprising, in each unit, substances in amounts meeting the following proportional relationship:
2.2 g of isoleucine, 3.4 g of leucine, 2.65 g of lysine acetate (equivalent to 1.8775 g of lysine), 0.3 g of methionine, 0.4 g of phenylalanine, 1.15 g of threonine, 0.375 g of tryptophan, 2.65 g of valine, 2.2 g of arginine, 1.175 g of histidine, 1.575 g of glycine, 2.075 g of alanine, 1.775 g of proline, 0.625 g of aspartic acid, 0.1375 g of asparagine, 0.2 g of N-acetyl-L-cysteine (equivalent to 0.15 g of cysteine), 0 or 1.425 g of glutamic acid, 0.415 g of L-ornithine hydrochloride (equivalent to 0.325 g of L-ornithine), 0.925 g of serine, 0.215 g of N-acetyl-L-tyrosine (equivalent to 0.175 g of tyrosine), 3.0-5.0 g of vitamin B₆, 10% KCl 0 or 5-10ml, and 0 or 2.0-6.0 g of vitamin C; or
4.4 g of isoleucine, 6.8 g of leucine, 5.3 g of lysine acetate (equivalent to 3.775 g of lysine), 0.6 g of methionine, 0.8 g of phenylalanine, 2.3 g of threonine, 0.75 g of tryptophan, 5.3 g of valine, 4.4 g of arginine, 2.35 g of histidine, 3.15 g of glycine, 4.15 g of alanine, 3.55 g of proline, 1.25 g of aspartic acid, 0.275 g of asparagine, 0.4 g of N-acetyl-L-cysteine (equivalent to 0.3 g of cysteine), 0 or 2.85 g of glutamic acid, 0.83 g of L-ornithine hydrochloride (equivalent to 0.65 g of L-ornithine), 1.85 g of serine, 0.43 g of N-acetyl-L-tyrosine (equivalent to 0.35 g of tyrosine), 3.0-10 g of vitamin B₆, 0 or 5-10 ml of 10% KCl, and 0 or 2.0-6.0 g of vitamin C.

5. The composition for use according to any of claims 1-4, having a formula comprising specifically:
250 ml of 0.9% sodium chloride injection + 3.0 g or 5.0 g of vitamin B₆ + 250 ml or 500 ml of compound amino acid injection;
250 ml of 0.9% sodium chloride injection + 3.0 g or 5.0 g of vitamin B₆ + 250 ml or 500 ml of compound amino acid injection + 2.0 g of vitamin C;
250 ml of 0.9% sodium chloride injection + 3.0 g or 5.0 g of vitamin B₆ + 250 ml or 500 ml of compound amino acid injection + 5 ml of 10% potassium chloride;
250 ml of 0.9% sodium chloride injection + 3.0 g or 5.0 g of vitamin B₆ + 250 ml or 500 ml of compound amino acid injection + 2.0 g of vitamin C + 5 ml of 10% potassium chloride;
250 ml of 5% glucose and sodium chloride injection+ 4u insulin injection + 3.0 g or 5.0 g of vitamin B₆ + 2.0 g of vitamin C + 5 ml of 10% potassium chloride + 250 ml of compound amino acid injection; or
250 ml of 10% glucose injection + 8u insulin injection+ 3.0 g or 5.0 g of vitamin B₆ + 2.0 g of vitamin C + 5 ml of 10% potassium chloride + 250 ml of compound amino acid injection,
wherein
the compound amino acid injection comprises, in every 1000 ml, 8.80 g of isoleucine, 13.60 g of leucine, 10.60 g of lysine acetate (equivalent to 7.51 g of lysine), 1.20 g of methionine, 1.60 g of phenylalanine, 4.60 g of threonine, 1.50 g of tryptophan, 10.60 g of valine, 8.80 g of arginine, 4.70 g of histidine, 6.30 g of glycine, 8.30 g of alanine, 7.10 g of proline, 2.50 g of aspartic acid, 0.55 g of asparagine, 0.80 g of N-acetyl-L-cysteine (equivalent to 0.60 g of cysteine), 5.70 g of glutamic acid, 1.66 g of L-ornithine hydrochloride (equivalent to 1.30 g of L-ornithine ), 3.70 g of serine, and 0.86 g of N-acetyl-L-tyrosine (equivalent to 0.70 g of tyrosine).

6. The composition for use according to any one of claims 1 to 5, which is in a dosage form selected from injectable solutions, oral liquids, tablets, granules, capsules, and instant soluble powders.

7. The composition for use according to any of claims 1-5, wherein the MND is amyotrophic lateral sclerosis.

8. The composition for use according to any of claims 5-6, wherein said method comprises giving the composition to a patient by injection or oral administration, wherein the injection is preferably intravenous injection.

9. The composition for use according to claim 8, wherein the patient is given the composition by injection or oral administration for 10-40 days and preferably 15-30 days in each course of treatment, and wherein, optionally, after one course of treatment is ended, a next course of treatment is proceeded after an appropriate break time as desired.

10. The composition for use according to claim 9, wherein a glucose injection and insulin are supplemented for patients with dysphagia, cough, or eating problem.

## Patentansprüche

1. Zusammensetzung zur Verwendung in einem Verfahren zur Behandlung von Motoneuronenerkrankungen (MNDs), wobei die MNDs amyotrophe Lateralsklerose, spinale Muskelatrophie, primäre Lateralsklerose oder progressive Bulbärparese sind, wobei die Zusammensetzung in jeder Einheit Substanzen in Mengen umfasst, die dem folgenden proportionalen Verhältnis entsprechen, wobei sich "Einheit" auf die einem Patienten verabreichte Tagesdosis bezieht:
03 bis 8 g an L-Ornithin oder eines L-Ornithinsalzes mit einem L-Ornithingehalt, der 0,3 bis 8 g entspricht, 0,5 bis 5 g Asparaginsäure und 2 bis 20 g Vitamin B₆;
vorzugsweise 0,325 bis 8 g an L-Ornithin oder eines L-Ornithinsalzes mit einem L-Ornithingehalt, der 0,325 bis 8 g entspricht, 0,625 bis 5 g Asparaginsäure und 3 bis 20 g Vitamin B₆;
ferner vorzugsweise 0,5 bis 8 g an L-Ornithin oder eines L-Ornithinsalzes mit einem L-Ornithingehalt, der 0,5 bis 8 g entspricht, 1 bis 5 g Asparaginsäure und 6 bis 20 g Vitamin B₆;
oder
ferner vorzugsweise 0,325 bis 0,65 g an L-Ornithin oder eines L-Ornithinsalzes mit einem L-Ornithingehalt, der 0,325 bis 0,65 g entspricht, 0,625 bis 1,25 g Asparaginsäure und 3 bis 10 g Vitamin B₆.

2. Zusammensetzung zur Verwendung nach Anspruch 1, ferner umfassend in jeder Einheit eines oder mehrere von Arginin, Isoleucin, Leucin, Lysin, Methionin, Phenylalanin, Threonin, Tryptophan, Valin, Histidin, Glycin, Alanin, Prolin, Asparagin, Cystein, Glutaminsäure, Serin, Tyrosin, Vitamin B₁, Vitamin B₂, Vitamin B₃, Pantothensäure, Biotin, Folsäure, Vitamin B₁₂, Vitamin C und KCl, wobei
die Aminosäuren wahlweise durch verschiedene lösliche Salze oder Derivate davon ersetzt sind;
wobei das Lysin durch Lysinacetat ersetzt ist, das Cystein durch N-Acetyl-L-Cystein ersetzt ist und das Tyrosin durch N-Acetyl-L-Tyrosin ersetzt ist, wobei die eine oder mehrere Substanzen in Mengen verwendet werden, die dem folgenden proportionalen Verhältnis entsprechen:
die Aminosäuren in Mengen verwendet werden, die dem proportionalen Verhältnis entsprechen von: 2,0-10 g Arginin, 2,0-10 g Isoleucin, 3,0-15 g Leucin, Lysinacetat mit einem Lysingehalt, der 1,5-10 g entspricht, 0,2-3 g Methionin, 0,3-3 g Phenylalanin, 1,0-10 g Threonin, 0,3-3 g Tryptophan, 2,5-15 g Valin, 1,0-8 g Histidin, 1,5-8 g Glycin, 2,0-10 g Alanin, 1,5-8 g Prolin, 0,1-3 g Asparagin, N-Acetyl-L-Cystein mit einem Cysteingehalt, der 0,1-3 g entspricht, 1,0-10 g Glutaminsäure, 0,5-5 g Serin und N-Acetyl-L-Tyrosin mit einem Tyrosingehalt, der 0,1-3 g entspricht;
die Vitamine in Mengen verwendet werden, die dem proportionalen Verhältnis entsprechen von: 1,0-4,0 mg Vitamin B₁, 1,0-4,0 mg Vitamin B₂, 10-40 mg Vitamin B₃, 3,0-10 mg Pantothensäure, 0,1-0,4 mg Biotin, 0,1-0,8 mg Folsäure, 2,0-12 µg Vitamin B₁₂ und 1,0-6,0 g Vitamin C; und
KCl in einer Menge von 5-10 ml 10% KCl verwendet wird.

3. Zusammensetzung zur Verwendung nach Anspruch 2, wobei die Aminosäuren in Mengen verwendet werden, die dem proportionalen Verhältnis entsprechen von: 2,2-4,4 g Arginin, 2,2-4,4 g Isoleucin, 3,4-6,8 g Leucin, Lysinacetat mit einem Lysingehalt, der 1,8775-3,755 g entspricht, 0,3-0,6 g Methionin, 0,4-0,8 g Phenylalanin, 1,15-2,3 g Threonin, 0,375-0,75 g Tryptophan, 2,65-5,3 g Valin, 1,175-2,35 g Histidin, 1,575-3,15 g Glycin, 2,075-4,15 g Alanin, 1,775-3,55 g Prolin, 0,1375-0,275 g Asparagin, N-Acetyl-L-Cystein mit einem Cysteingehalt, der 0,15-0,3 g entspricht, 1,425-2,85 g Glutaminsäure, 0,925-1,85 g Serin und N-Acetyl-L-Tyrosin mit einem Tyrosingehalt, der 0,175-0,35 g entspricht;
die Vitamine in Mengen verwendet werden, die dem proportionalen Verhältnis entsprechen von: 1,0-4,0 mg Vitamin B₁, 1,0-4,0 mg Vitamin B₂, 10-40 mg Vitamin B₃, 3,0-10 mg Pantothensäure, 0,1-0,4 mg Biotin, 0,1-0,8 mg Folsäure, 2,0-12 µg Vitamin B₁₂ und 1,0-6,0 g Vitamin C; und
KCl in einer Menge von 5-10 ml 10% KCl verwendet wird; oder
die Aminosäuren in Mengen verwendet werden, die dem proportionalen Verhältnis entsprechen von: 3,0-10 g Arginin, 3,0-10 g Isoleucin, 5,0-15 g Leucin, Lysinacetat mit einem Lysingehalt, der 3,0-10 g entspricht, 0,5-3 g Methionin, 0,5-3 g Phenylalanin, 3,0-10 g Threonin, 0,5-3,0 g Tryptophan, 5,0-15 g Valin, 3,0-8,0 g Histidin, 3,0-8,0 g Glycin, 3,0-10 g Alanin, 3,0-8,0g Prolin, 0,1-3,0 g Asparagin, N-Acetyl-L-Cystein mit einem Cysteingehalt, der 0,1-3,0 g entspricht, 3,0-10 g Glutaminsäure, 0,5-5,0 g Serin und N-Acetyl-L-Tyrosin mit einem Tyrosingehalt, der 0,1-3 g entspricht;
die Vitamine in Mengen verwendet werden, die dem proportionalen Verhältnis entsprechen von: 2,0-4,0 mg Vitamin B₁, 2,0-4,0 mg Vitamin B₂, 20-40 mg Vitamin B₃, 6,0-10 mg Pantothensäure, 0,2-0,4 mg Biotin, 0,2-0,8 mg Folsäure, 4,0-12 µg Vitamin B₁₂ und 2,0-6,0 g Vitamin C.

4. Zusammensetzung zur Verwendung nach einem der Ansprüche 2-3, die in jeder Einheit Substanzen in Mengen umfasst, die dem folgenden proportionalen Verhältnis entsprechen:
2,2 g Isoleucin, 3,4 g Leucin, 2,65 g Lysinacetat (entsprechend 1,8775 g Lysin), 0,3 g Methionin, 0,4 g Phenylalanin, 1,15 g Threonin, 0,375 g Tryptophan, 2,65 g Valin, 2,2 g Arginin, 1,175 g Histidin, 1,575 g Glycin, 2,075 g Alanin, 1,775 g Prolin, 0,625 g Asparaginsäure, 0,1375 g Asparagin, 0,2 g N-Acetyl-L-Cystein (entsprechend 0,15 g Cystein), 0 oder 1,425 g Glutaminsäure, 0,415 g L-Ornithinhydrochlorid (entsprechend 0,325 g L-Ornithin), 0,925 g Serin, 0,215 g N-Acetyl-L-Tyrosin (entspricht 0,175 g Tyrosin), 3,0-5,0 g Vitamin B₆, 10% KCl 0 oder 5-10 ml und 0 oder 2,0-6,0 g Vitamin C; oder
4,4 g Isoleucin, 6,8 g Leucin, 5,3 g Lysinacetat (entsprechend 3,775 g Lysin), 0,6 g Methionin, 0,8 g Phenylalanin, 2,3 g Threonin, 0,75 g Tryptophan, 5,3 g Valin, 4,4 g Arginin, 2,35 g Histidin, 3,15 g Glycin, 4,15 g Alanin, 3,55 g Prolin, 1,25 g Asparaginsäure, 0,275 g Asparagin, 0,4 g N-Acetyl-L-Cystein (entsprechend 0,3 g Cystein), 0 oder 2,85 g Glutaminsäure, 0,83 g L-Ornithinhydrochlorid (entsprechend 0,65 g L-Ornithin), 1,85 g Serin, 0,43 g N-Acetyl-L-Tyrosin (entsprechend 0,35 g Tyrosin), 3,0-10,0 g Vitamin B₆, 0 oder 5-10 ml 10% KCl und 0 oder 2,0-6,0 g Vitamin C.

5. Zusammensetzung zur Verwendung nach einem der Ansprüche 1-4, mit einer Formulierung, die insbesondere umfasst:
250 ml 0,9%ige Natriumchlorid-Injektion + 3,0 g oder 5,0 g Vitamin B₆ + 250 ml oder 500 ml Aminosäureverbindungs-Injektion;
250 ml 0,9%ige Natriumchlorid-Injektion + 3,0 g oder 5,0 g Vitamin B₆ + 250 ml oder 500 ml Aminosäureverbindungs-Injektion + 2,0 g Vitamin C;
250 ml 0,9%ige Natriumchlorid-Injektion + 3,0 g oder 5,0 g Vitamin B₆ + 250 ml oder 500 ml Aminosäureverbindungs-Injektion + 5 ml 10%iges Kaliumchlorid;
250 ml 0,9%ige Natriumchlorid-Injektion + 3,0 g oder 5,0 g Vitamin B₆ + 250 ml oder 500 ml Aminosäureverbindungs-Injektion + 2,0 g Vitamin C + 5 ml 10%iges Kaliumchlorid;
250 ml 5%ige Glukose- und Natriumchlorid-Injektion + 4u Insulin-Injektion + 3,0 g oder 5,0 g Vitamin B₆ + 2,0 g Vitamin C + 5 ml 10%iges Kaliumchlorid + 250 ml Aminosäureverbindungs-Injektion; oder
250 ml 10 %ige Glukose-Injektion + 8u Insulin-Injektion + 3,0 g oder 5,0 g Vitamin B₆ + 2,0 g Vitamin C + 5 ml 10 %iges Kaliumchlorid + 250 ml Aminosäureverbindungs-Injektion,
wobei
die Aminosäureverbindungs-Injektion je 1000 ml 8,80 g Isoleucin, 13,60 g Leucin, 10,60 g Lysinacetat (entsprechend 7,51 g Lysin), 1,20 g Methionin, 1,60 g Phenylalanin, 4,60 g Threonin, 1,50 g Tryptophan, 10,60 g Valin, 8,80 g Arginin, 4,70 g Histidin, 6,30 g Glycin, 8,30 g Alanin, 7,10 g Prolin, 2,50 g Asparaginsäure, 0,55 g Asparagin, 0,80 g N-Acetyl-L-Cystein (entsprechend 0,60 g Cystein), 5,70 g Glutaminsäure, 1,66 g L-Ornithinhydrochlorid (entsprechend 1,30 g L-Ornithin), 3,70 g Serin und 0,86 g N-Acetyl-L-Tyrosin (entsprechend 0,70 g Tyrosin) umfasst.

6. Zusammensetzung zur Verwendung nach einem der Ansprüche 1 bis 5, die in einer Darreichungsform vorliegt, ausgewählt aus injizierbaren Lösungen, oralen Flüssigkeiten, Tabletten, Granulaten, Kapseln und sofort löslichen Pulvern.

7. Zusammensetzung zur Verwendung nach einem der Ansprüche 1-5, wobei die MND amyotrophe Lateralsklerose ist.

8. Zusammensetzung zur Verwendung nach einem der Ansprüche 5-6, wobei das Verfahren die Verabreichung der Zusammensetzung an einen Patienten mittels Injektion oder oraler Verabreichung umfasst, wobei die Injektion vorzugsweise intravenöse Injektion ist.

9. Zusammensetzung zur Verwendung nach Anspruch 8, wobei dem Patienten die Zusammensetzung mittels Injektion oder oraler Verabreichung für 10-40 Tage und vorzugsweise 15-30 Tage in jedem Behandlungsdurchgang verabreicht wird, und wobei, wahlweise, nach Beendigung eines Behandlungsdurchgangs, ein nächster Behandlungsdurchgang nach einer angemessenen Pausenzeit, wie gewünscht, fortgesetzt wird.

10. Zusammensetzung zur Verwendung nach Anspruch 9, wobei eine Glukose-Injektion und Insulin für Patienten mit Dysphagie, Husten oder Essproblemen ergänzt werden.

## Revendications

1. Composition destinée à être utilisée dans un procédé de traitement des maladies des motoneurones (MND), les MND étant la sclérose latérale amyotrophique, l'amyotrophie spinale, la sclérose latérale primaire ou la paralysie bulbaire progressive, ladite composition comprenant, dans chaque unité, des substances en quantités satisfaisant la relation proportionnelle suivante, « unité » faisant référence à la dose quotidienne administrée à un patient :
0,3 à 8 g de L-ornithine ou d'un sel de L-ornithine avec une teneur en L-ornithine équivalente à 0,3 à 8 g, 0,5 à 5 g d'acide aspartique et 2 à 20 g de vitamine B₆ ;
de préférence 0,325 à 8 g de L-ornithine ou d'un sel de L-ornithine avec une teneur en L-ornithine équivalente à 0,325 à 8 g, 0,625 à 5 g d'acide aspartique et 3 à 20 g de vitamine B₆ ;
plus préférablement 0,5 à 8 g de L-ornithine ou d'un sel de L-ornithine ayant une teneur en L-ornithine équivalente à 0,5 à 8 g, 1 à 5 g d'acide aspartique et 6 à 20 g de vitamine B₆ ;
ou
plus préférablement 0,325 à 0,65 g de L-ornithine ou d'un sel de L-ornithine ayant une teneur en L-ornithine équivalente à 0,325 à 0,65 g, 0,625 à 1,25 g d'acide aspartique et 3 à 10 g de vitamine B₆.

2. Composition pour utilisation selon la revendication 1, comprenant en outre, dans chaque unité, l'un ou plusieurs parmi l'arginine, l'isoleucine, la leucine, la lysine, la méthionine, la phénylalanine, la thréonine, le tryptophane, la valine, l'histidine, la glycine, l'alanine, la proline, l'asparagine, la cystéine, l'acide glutamique, la sérine, la tyrosine, la vitamine B₁, la vitamine B₂, la vitamine B₃, l'acide pantothénique, la biotine, l'acide folique, la vitamine B₁₂, la vitamine C et KCl, dans laquelle
les acides aminés sont éventuellement remplacés par différents sels solubles ou dérivés de ceux-ci ;
dans laquelle la lysine est remplacée par l'acétate de lysine, la cystéine est remplacée par la N-acétyl-L-cystéine et la tyrosine est remplacée par la N-acétyl-L-tyrosine, dans laquelle les une ou plusieurs substances sont utilisées en quantités satisfaisant la relation proportionnelle suivante :
les acides aminés étant utilisés en quantités satisfaisant la relation proportionnelle de 2,0 à 10 g d'arginine, 2,0 à 10 g d'isoleucine, 3,0 à 15 g de leucine, de l'acétate de lysine avec une teneur en lysine équivalente à 1,5 à 10 g, 0,2 à 3 g de méthionine, 0,3 à 3 g de phénylalanine, 1,0 à 10 g de thréonine, 0,3 à 3 g de tryptophane, 2,5 à 15 g de valine, 1,0 à 8 g d'histidine, 1,5 à 8 g de glycine, 2,0 à 10 g d'alanine, 1,5 à 8 g de proline, 0,1 à 3 g d'asparagine, de la N-acétyl-L-cystéine avec une teneur en cystéine équivalente à 0,1 à 3 g, 1,0 à 10 g d'acide glutamique, 0,5 à 5 g de sérine et de la N-acétyl-L-tyrosine avec une teneur en tyrosine équivalente à 0,1 à 3 g ;
les vitamines étant utilisées en quantités satisfaisant la relation proportionnelle de 1,0 à 4,0 mg de vitamine B₄, 1,0 à 4,0 mg de vitamine B₂, 10 à 40 mg de vitamine B₃, 3,0 à 10 mg d'acide pantothénique, 0,1 à 0,4 mg de biotine, 0,1 à 0,8 mg d'acide folique, 2,0 à 12 µg de vitamine B₁₂ et 1,0 à 6,0 g de vitamine C ; et
KCI étant utilisé en une quantité de 5 à 10 ml de KCI à 10 %.

3. Composition pour utilisation selon la revendication 2, dans laquelle les acides aminés sont utilisés en quantités satisfaisant la relation proportionnelle de 2,2 à 4,4 g d'arginine, 2,2 à 4,4 g d'isoleucine, 3,4 à 6,8 g de leucine, de l'acétate de lysine avec une teneur en lysine équivalente à 1,8775 à 3,755 g, 0,3 à 0,6 g de méthionine, 0,4 à 0,8 g de phénylalanine, 1,15 à 2,3 g de thréonine, 0,375 à 0,75 g de tryptophane, 2,65 à 5,3 g de valine, 1,175 à 2,35 g d'histidine, 1,575 à 3,15 g de glycine, 2,075 à 4,15 g d'alanine, 1,775 à 3,55 g de proline, 0,1375 à 0,275 g d'asparagine, de N-acétyl-L-cystéine avec une teneur en cystéine équivalente à 0,15 à 0,3 g, de 1,425 à 2,85 g d'acide glutamique, de 0,925 à 1,85 g de sérine et de N-acétyl-L-tyrosine avec une teneur en tyrosine équivalente à 0,175 à 0,35 g ;
les vitamines étant utilisées en quantités satisfaisant la relation proportionnelle de 1,0 à 4,0 mg de vitamine B₄, 1,0 à 4,0 mg de vitamine B₂, 10 à 40 mg de vitamine B₃, 3,0 à 10 mg d'acide pantothénique, 0,1 à 0,4 mg de biotine, 0,1 à 0,8 mg d'acide folique, 2,0 à 12 µg de vitamine B₁₂ et 1,0 à 6,0 g de vitamine C ; et
KCI étant utilisé en une quantité de 5 à 10 ml de KCI à 10 % ; ou
les acides aminés étant utilisés en quantités satisfaisant la relation proportionnelle de 3,0 à 10 g d'arginine, 3,0 à 10 g d'isoleucine, 5,0 à 15 g de leucine, de l'acétate de lysine avec une teneur en lysine équivalente à 3,0 à 10 g, 0,5 à 3 g de méthionine, 0,5 à 3 g de phénylalanine, 3,0 à 10 g de thréonine, 0,5 à 3,0 g de tryptophane, 5,0 à 15 g de valine, 3,0 à 8,0 g d'histidine, 3,0 à 8,0 g de glycine, 3,0 à 10 g d'alanine, 3,0 à 8,0 g de proline, 0,1 à 3,0 g d'asparagine, de la N-acétyl-L-cystéine avec une teneur en cystéine équivalente à 0,1 à 3,0 g, 3,0 à 10 g d'acide glutamique, 0,5 à 5,0 g de sérine et de la N-acétyl-L-tyrosine avec une teneur en tyrosine équivalente à 0,1 à 3 g ;
les vitamines étant utilisées en quantités satisfaisant la relation proportionnelle de 2,0 à 4,0 mg de vitamine B₁, 2,0 à 4,0 mg de vitamine B₂, 20 à 40 mg de vitamine B₃, 6,0 à 10 mg d'acide pantothénique, 0,2 à 0,4 mg de biotine, 0,2 à 0,8 mg d'acide folique, 4,0 à 12 µg de vitamine B₁₂ et 2,0 à 6,0 g de vitamine C.

4. Composition pour utilisation selon l'une quelconque des revendications 2 à 3, comprenant, dans chaque unité, des substances en quantités satisfaisant la relation proportionnelle suivante :
2,2 g d'isoleucine, 3,4 g de leucine, 2,65 g d'acétate de lysine (équivalent à 1,8775 g de lysine), 0,3 g de méthionine, 0,4 g de phénylalanine, 1,15 g de thréonine, 0,375 g de tryptophane, 2,65 g de valine, 2,2 g d'arginine, 1,175 g d'histidine, 1,575 g de glycine, 2,075 g d'alanine, 1,775 g de proline, 0,625 g d'acide aspartique, 0,1375 g d'asparagine, 0,2 g de N-acétyl-L-cystéine (équivalent à 0,15 g de cystéine), 0 ou 1,425 g d'acide glutamique, 0,415 g de chlorhydrate de L-ornithine (équivalent à 0,325 g de L-ornithine), 0,925 g de sérine, 0,215 g de N-acétyl-L-tyrosine (équivalent à 0,175 g de tyrosine), 3,0 à 5,0 g de vitamine B₆, 0 ou 5 à 10 ml de KCI à 10 % et 0 ou 2,0 à 6,0 g de vitamine C ; ou
4,4 g d'isoleucine, 6,8 g de leucine, 5,3 g d'acétate de lysine (équivalent à 3,775 g de lysine), 0,6 g de méthionine, 0,8 g de phénylalanine, 2,3 g de thréonine, 0,75 g de tryptophane, 5,3 g de valine, 4,4 g d'arginine, 2,35 g d'histidine, 3,15 g de glycine, 4,15 g d'alanine, 3,55 g de proline, 1,25 g d'acide aspartique, 0,275 g d'asparagine, 0,4 g de N-acétyl-L-cystéine (équivalent à 0,3 g de cystéine), 0 ou 2,85 g d'acide glutamique, 0,83 g de chlorhydrate de L-ornithine (équivalent à 0,65 g de L-ornithine), 1,85 g de sérine, 0,43 g de N-acétyl-L-tyrosine (équivalent à 0,35 g de tyrosine), 3,0 à 10 g de vitamine B₆, 0 ou 5 à 10 ml de KCI à 10 % et 0 ou 2,0 à 6,0 g de vitamine C.

5. Composition pour utilisation selon l'une quelconque des revendications 1 à 4, ayant une formule comprenant spécifiquement :
250 ml de solution injectable de chlorure de sodium à 0,9 % + 3,0 g ou 5,0 g de vitamine B₆ + 250 ml ou 500 ml de solution injectable de mélange d'acides aminés ;
250 ml de solution injectable de chlorure de sodium à 0,9 % + 3,0 g ou 5,0 g de vitamine B₆ + 250 ml ou 500 ml de solution injectable de mélange d'acides aminés + 2,0 g de vitamine C ;
250 ml de solution injectable de chlorure de sodium à 0,9 % + 3,0 g ou 5,0 g de vitamine B₆ + 250 ml ou 500 ml de solution injectable de mélange d'acides aminés + 5 ml de chlorure de potassium à 10 % ;
250 ml de solution injectable de chlorure de sodium à 0,9 % + 3,0 g ou 5,0 g de vitamine B₆ + 250 ml ou 500 ml de solution injectable de mélange d'acides aminés + 2,0 g de vitamine C 5 ml de chlorure de potassium à 10 % ;
250 ml de solution injectable de glucose à 5 % et de chlorure de sodium + 4u de solution injectable d'insuline + 3,0 g ou 5,0 g de vitamine B₆ + 2,0 g de vitamine C + 5 ml de chlorure de potassium à 10 % + 250 ml de solution injectable de mélange d'acides aminés ; ou
250 ml de solution injectable de glucose à 10 % + 8u de solution injectable d'insuline + 3,0 g ou 5,0 g de vitamine B₆ + 2,0 g de vitamine C + 5 ml de chlorure de potassium à 10 % + 250 ml de solution injectable de mélange d'acides aminés,
dans laquelle
la solution injectable de mélange d'acides aminés comprend, dans chaque 1000 ml, 8,80 g d'isoleucine, 13,60 g de leucine, 10,60 g d'acétate de lysine (équivalent à 7,51 g de lysine), 1,20 g de méthionine, 1,60 g de phénylalanine, 4,60 g de thréonine, 1,50 g de tryptophane, 10,60 g de valine, 8,80 g d'arginine, 4,70 g d'histidine, 6,30 g de glycine, 8,30 g d'alanine, 7,10 g de proline, 2,50 g d'acide aspartique, 0,55 g d'asparagine, 0,80 g de N-acétyl-L-cystéine (équivalent à 0,60 g de cystéine), 5,70 g d'acide glutamique, 1,66 g de chlorhydrate de L-ornithine (équivalent à 1,30 g de L-ornithine), 3,70 g de sérine et 0,86 g de N-acétyl-L-tyrosine (équivalent à 0,70 g de tyrosine).

6. Composition pour utilisation selon l'une quelconque des revendications 1 à 5, qui est sous une forme posologique choisie parmi des solutions injectables, des liquides oraux, des comprimés, des granulés, des capsules et des poudres solubles instantanées.

7. Composition pour utilisation selon l'une quelconque des revendications 1 à 5, la MND étant la sclérose latérale amyotrophique.

8. Composition pour utilisation selon l'une quelconque des revendications 5 à 6, ledit procédé comprenant l'administration de la composition à un patient par injection ou administration orale, l'injection étant de préférence une injection intraveineuse.

9. Composition pour utilisation selon la revendication 8, le patient recevant la composition par injection ou administration orale pendant 10 à 40 jours et de préférence 15 à 30 jours dans chaque cure de traitement, et éventuellement, après la fin d'une cure de traitement, une cure de traitement suivante étant poursuivie après un temps de pause approprié comme souhaité.

10. Composition pour utilisation selon la revendication 9, une solution injectable de glucose et de l'insuline étant ajoutées pour les patients souffrant de dysphagie, de toux ou de problèmes alimentaires.
